# EUROPEAN PATENT APPLICATION

(11) **EP 4 578 503 A2**
(43) Date of publication of application: **02.07.2025**
(21) Application number: 25163101.6
(22) Date of filing: 09.07.2020
(51) Int. Cl.: A61P 21/00

(54) **NON-VIRAL IMMUNO-TARGETING**

(30) Priority: 10.07.2019 US 201962872425 P
(62) Divisional of application: 20836718.5
(71) Applicant: University of Washington, Seattle, WA 98105-4721 (US)
(72) Inventor: WHITEHEAD, Nicholas P., Seattle (US); FROEHNER, Stanley C., Seattle (US)
(74) Representative: Mathys & Squire

(57) **Abstract**

The disclosure provides compositions and methods for delivering a payload to cells or tissues that express GLUT4. In some embodiments, the compositions comprise an antibody, or fragment or derivative thereof, that specifically binds to glucose transporter 4 ("GLUT4") protein, and a therapeutic payload conjugated thereto. In some exemplary embodiments, the compositions are useful for methods of treating a disease or condition in a subject with a genetic mutation in a gene encoding dystrophin protein, wherein the payload comprises a nucleic acid encoding a functional dystrophin protein or functional fragment thereof to ameliorate aspects of the disease.

## Description

### CROSS-REFERENCES TO RELATED APPLICATIONS

This application claims priority benefit of U.S. provisional application no. 62/872,425, filed July 10, 2019, which is incorporated by reference for all purposes.

### STATEMENT REGARDING SEQUENCE LISTING

The sequence listing associated with this application is provided in text format in lieu of a paper copy and is hereby incorporated by reference into the specification. The name of the text file containing the sequence listing is 72245_SEQ_Final_2020-07-08.txt. The text file is 145 KB; was created on July 8, 2020; and is being submitted via EFS-Web with the filing of the specification.

### BACKGROUND

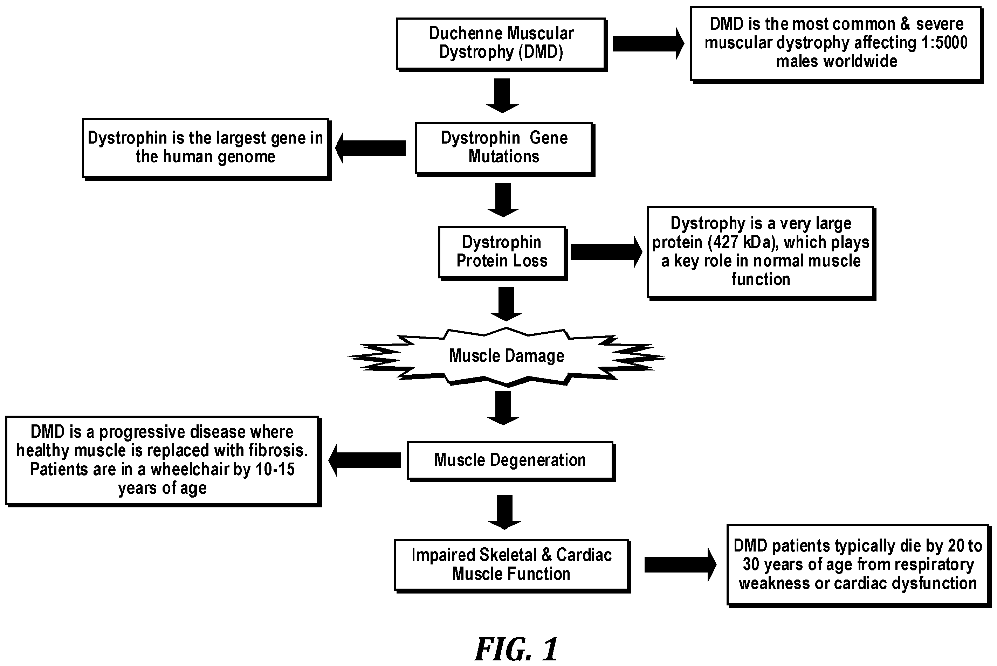

Human diseases involving muscle range from relatively rare conditions such as muscular dystrophies, to common ailments such as diabetes and age-related muscle weakness (sarcopenia). Currently, there are no effective treatments for many muscle diseases, including the most common and severe form of muscular dystrophy, known as Duchenne muscular dystrophy (DMD). A brief overview of the main features of DMD is shown schematically in Fig. 1. Since dystrophin, the gene responsible for DMD, was discovered in 1987, researchers have attempted to develop techniques to deliver the dystrophin gene to muscles and thereby improve or even cure the disease. However, a major drawback to this approach for DMD is the huge size of the wild-type dystrophin gene, ~14 kilobases for the mature transcript, which is far too large to fit into viral vectors available for delivering genes to tissues. To illustrate, the most commonly used gene therapy viral vector is adeno-associated virus (AAV), which has a total packaging capacity of only 5 kilobases. Consequently, to provide a deliverable therapeutic dystrophin transgene, the dystrophin gene must be truncated to 'micro-dystrophins', which are about one third the size of full-length dystrophin and provide only partial improvement of in vivo muscle function in the mdx (DMD) mouse. Other major problems associated with viral vectors include a lack of specificity in targeting the vector to the desired cell type and potential immune responses associated with viral delivery. The potential of eliciting an immune response is a significant limitation of AAV approaches because it functionally limits therapeutic delivery to a single administration. This is very likely to be insufficient for treating degenerative muscle diseases such as DMD, which can require intervention over years or even decades. In addition, many humans have already been exposed to certain AAV serotypes, which would exclude them from AAV gene therapy altogether.

Accordingly, despite the advances in the art of understanding muscular diseases and viral vectors for therapeutic payloads, a need remains for non-immunogenic vector platforms that can specifically deliver therapeutic payloads to muscle cells without limitations on payload size or type. This disclosure addresses these and related needs.

### SUMMARY

This summary is provided to introduce a selection of concepts in an abbreviated form that are further described below in the Detailed Description. This summary is not intended to identify key features of the claimed subject matter, nor is it intended to be used as an aid in determining the scope of the claimed subject matter.

In one aspect, the disclosure provides a composition comprising a binding domain, *e.g.,* an antibody, or fragment or derivative thereof, that specifically binds to glucose transporter 4 ("GLUT4") protein, and a therapeutic payload conjugated to the binding domain.

In another aspect, the disclosure provides a method of targeting a therapeutic payload for delivery to a cell that expresses GLUT4, the method comprising contacting the cell with the disclosed composition. The method can be *in vitro.* Alternatively, the method can be an *in vivo* method of treating a disease or condition associated with GLUT4 expressing cells.

In another aspect, the disclosure provides a method of treating a disease or condition in a subject with a genetic mutation in a gene encoding dystrophin protein, comprising administering to a subject a therapeutically effective amount of the disclosed composition. The composition in this method aspect comprises a binding domain, e.g., an antibody, or fragment or derivative thereof, that specifically binds glucose transporter 4 ("GLUT4") protein, and a therapeutic payload conjugated to the antibody, or fragment or derivative thereof. The therapeutic payload comprises a nucleic acid with a promoter sequence operatively linked to a sequence encoding a dystrophin protein, or a functional fragment thereof.

### BRIEF DESCRIPTION OF THE DRAWINGS

FIGURE 1 is a schematic overview of Duchenne muscular dystrophy (DMD).

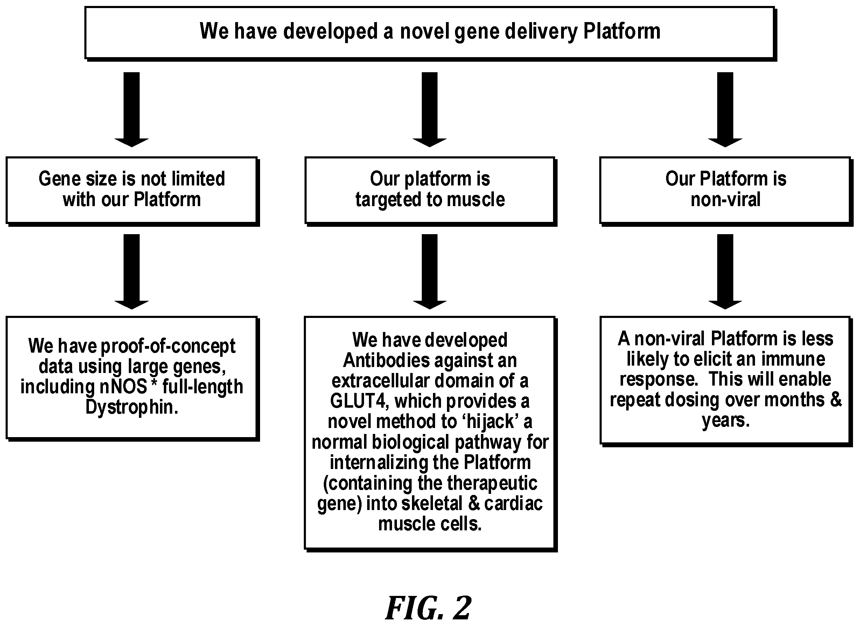

FIGURE 2 is a schematic overview of aspects of the non-viral, muscle-targeted gene delivery platform disclosed herein.

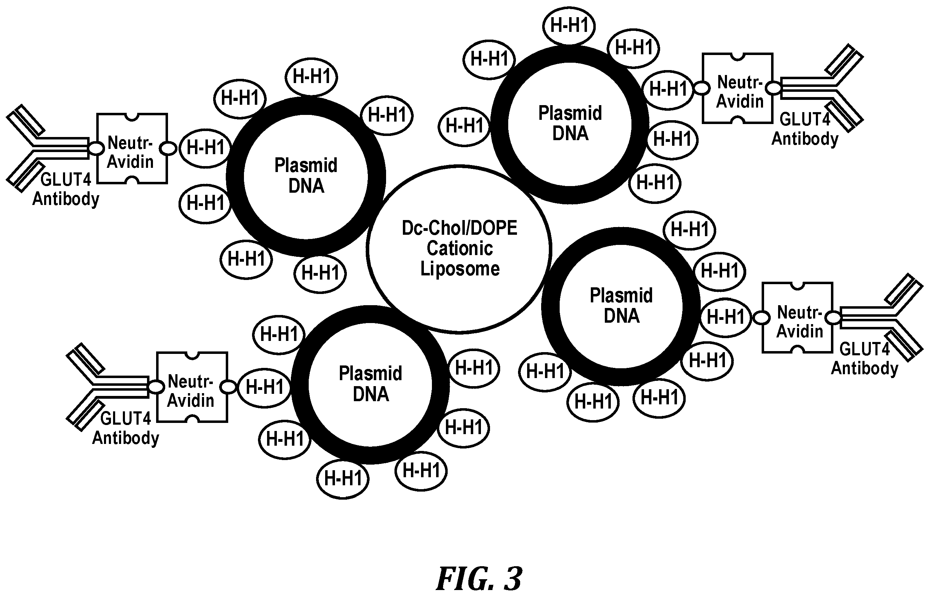

FIGURE 3 is a schematic of the general structure of a representative platform of the disclosed compositions that were used for *in vitro* cell culture experiments as described in more detail below.

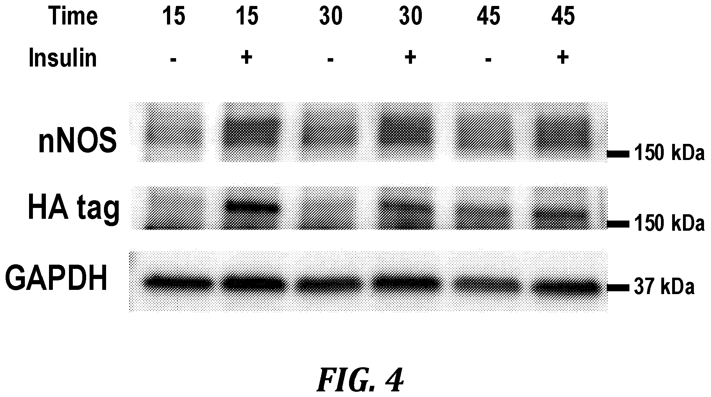

FIGURE 4 illustrates western blot results from Experiment 1, described below, showing expression of nNOS and HA tag. GAPDH was used as a protein loading control. Incubation of the platform with muscle cells for only 15 min are shown to lead to expression of nNOS.

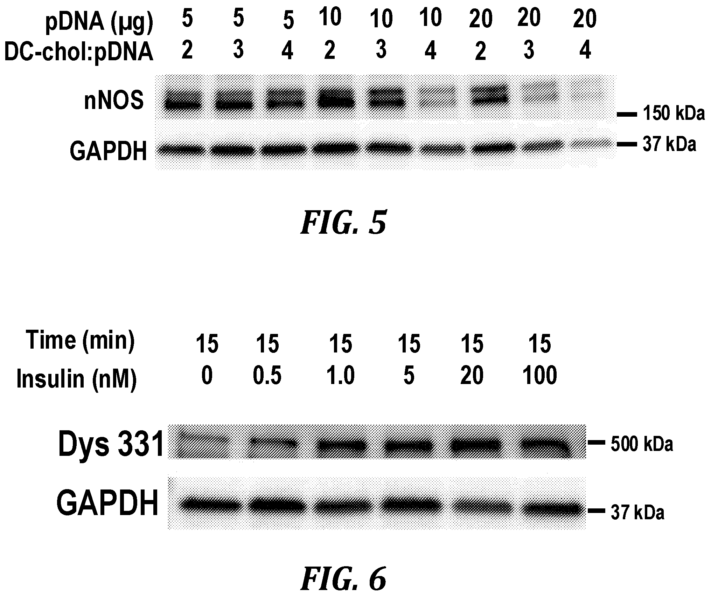

FIGURE 5 illustrates western blot results from Experiment 2, described below, showing expression of nNOS. GAPDH was used as a protein loading control. Here, the effect of different plasmid DNA concentrations (pDNA) and different ratios of DC-chol:pDNA were tested.
FIGURE 6 illustrates western blot results from Experiment 3, described below, showing expression of full-length Dystrophin using the antibody 331 (C-terminal antibody) with increasing concentrations of administered insulin. GAPDH was used as a protein loading control. The results indicated that adding insulin up to 5 nM increased the expression of Dystrophin.

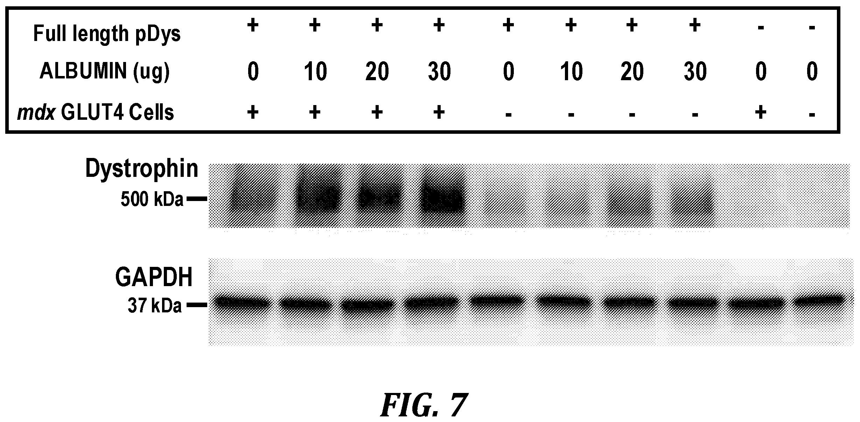

FIGURE 7 illustrates western blot results from Experiment 4, described below, showing expression of full-length Dystrophin using the antibody 331 (C-terminal antibody). GAPDH was used as a protein loading control. As shown, Dystrophin expression is higher in cells overexpressing (+) GLUT4 (*mdx* GLUT4), and increasing amounts of Albumin added to the expression.

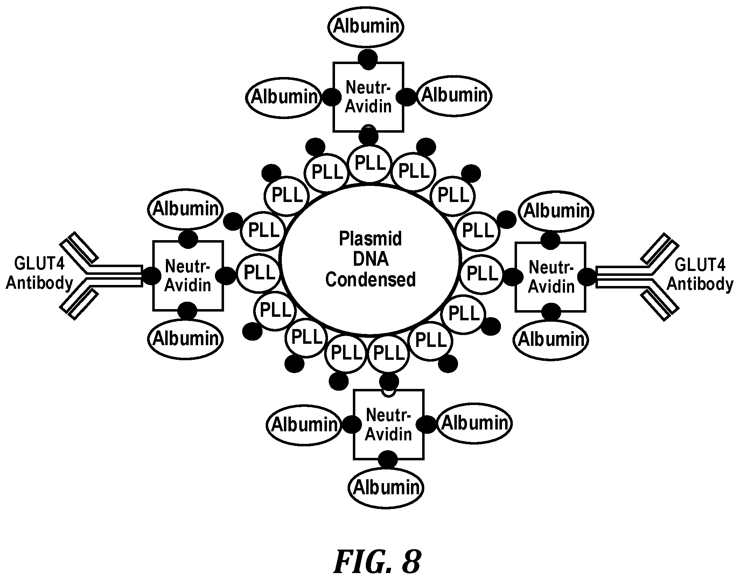

FIGURE 8 is a schematic of the general structure and components of another aspect of the disclosed composition, which was for the *in vivo* experiments as described in more detail below.

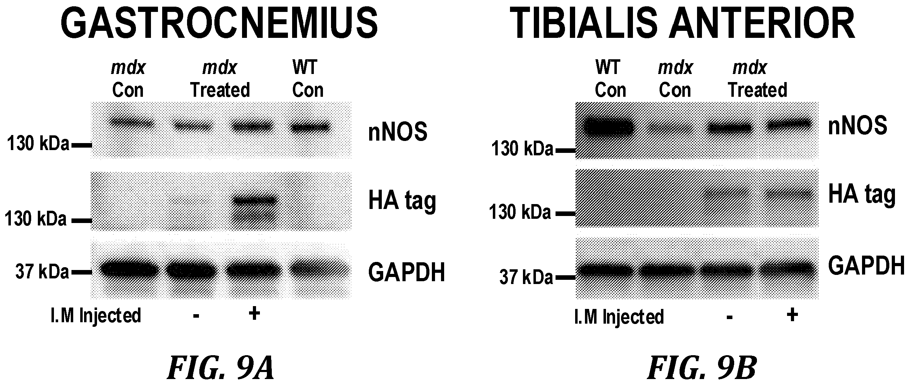

FIGURE 9A-B illustrates western blot results from Experiment 5, described below, showing expression of nNOS and HA tag following *in vivo* administration of the platform without the GLUT4 antibody added. GAPDH was used as a protein loading control. Increased expression of both nNOS and the HA tag was observed in Gastrocnemius (9A) and Tibialis Anterior (9B) muscles of the treated mouse compared to the untreated *mdx* Con mouse. Both the injected (+) and noninjected (-) muscles show expression of nNOS, indicating that the composition was able to leak out of the injected muscles and achieve systemic circulation to ultimately transfect muscles in the contralateral (noninjected) limb.

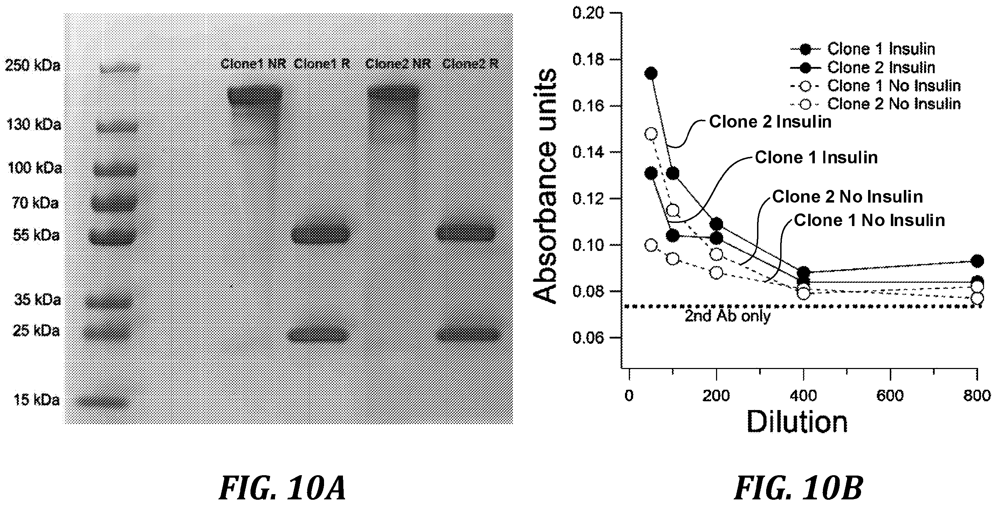

FIGURE 10A-B. (10A) Coomassie stain showing the successful purification of two GLUT4 mAb clones using protein A/G columns. The coomassie stain of each clone is shown under non-reducing (NR) or reducing (R) conditions; (10B) graphically illustrates binding of mAb clones to native GLUT4 protein with and without insulin. *Mdx* myoblasts overexpressing GLUT4 were incubated with mouse mAb clones 1 & 2 (at different dilutions of a 1 mg/ml stock solution), with or without 100 nM insulin, for 20 min at 37°C followed by a HRP-conjugated secondary antibody for the same time. Antibody binding (Absorbance) was measured using a colorimetric assay. The increased Absorbance shows that both clones bind to the native GLUT4 protein *in vitro,* with Clone 2 showing greater binding affinity than Clone 1. For both clones, insulin increases the Absorbance by trafficking more GLUT4 to the plasma membrane. The Absorbance of a secondary antibody only control is shown (dotted line).

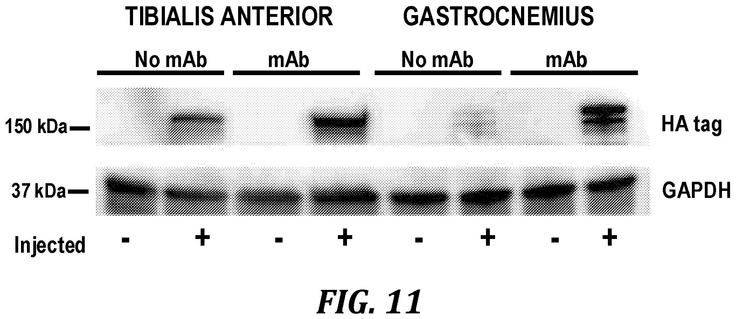

FIGURE 11 illustrates western blot results from Experiment 6, described below, showing expression of the HA tag following *in vivo* administration (I.M. injection into the TA and Gastrocnemius) of the platform composition without ("No mAb") or with ("mAb") the GLUT4 antibody added. GAPDH was used as a protein loading control. A several fold increase in HA tag expression was observed in both muscles of the mouse treated with the mAb-conjugated platform composition.

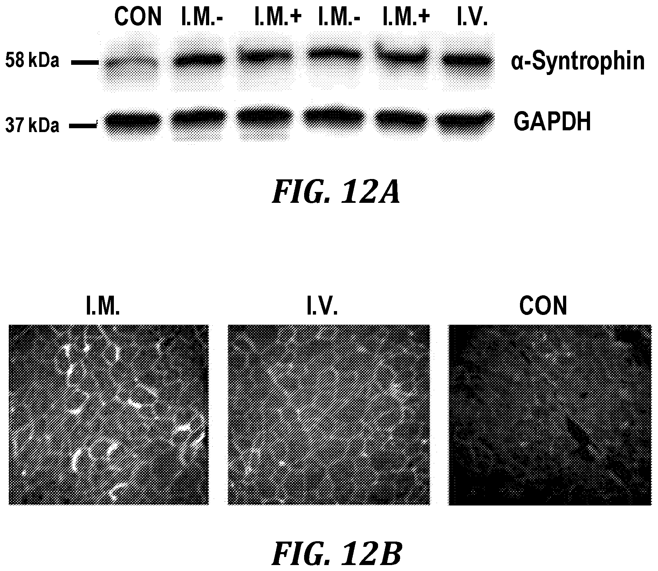

FIGURE 12A-B illustrates results from Experiment 7, showing expression of α-syntrophin in *mdx*4CV mice following *in vivo* administration (I.M. injection into the TA and Gastrocnemius or I.V. injection via the retro-orbital sinus) of the platform composition containing the GLUT4 antibody (see Fig. 8). The α-syntrophin DNA construct contained a RAS palmitoylation sequence for targeting the protein to the muscle surface membrane (sarcolemma). (12A) Western blot results show much greater expression of α-syntrophin in muscles of both I.M. and I.V. injected mice compared to a non-injected control *mdx*4CV mouse. GAPDH was used as a protein loading control. (12B) Immunostaining of muscle cross-sections showing many muscle fibers with surface membrane localized α-syntrophin staining in both I.M. and I.V. injected mice, which is not evident in the non-injected control mouse. Note, the very bright areas of staining in some muscle fibers of the I.M. injected mouse are most likely neuromuscular junctions, which have more membrane surface area for α-syntrophin expression.

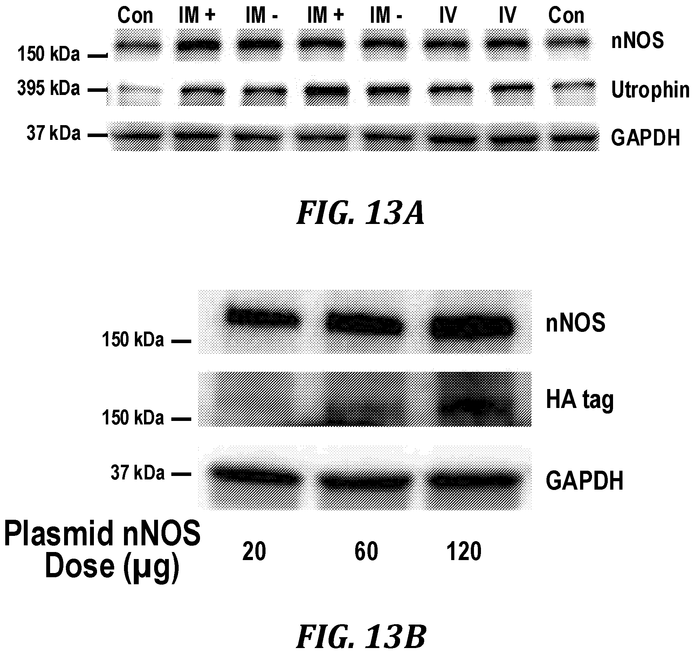

FIGURE 13A-B illustrates western blot results from Experiment 8, showing expression of nNOS in *mdx4CV* mice following *in vivo* administration of the platform containing the nNOS construct described in Fig. 11. (13A) Here, two mice were injected I.M. in the gastrocnemius muscle of one hindlimb and two mice were injected 1.V. via the retro-orbital sinus. The Western blot shows that both I.M. and I.V. injections led to a considerable increase in nNOS expression in the gastrocnemius compared to two gastrocnemius muscles from a non-injected *mdx*4CV control mouse. Consistent with previous experiments, the I.M. injection resulted in increased nNOS expression in muscles of both the injected (I.M.+) and contralateral (I.M.-) legs, indicating a systemic distribution of the platform following I.M. delivery. In addition, the dystrophin homologue, utrophin, was also considerably increased in the muscles of mice injected with the platform. GAPDH was used as a loading control. (13B) Three *mdx* mice were injected I.V. with different amounts of nNOS plasmid (20, 60 or 120 µg). As shown by Western blot, both nNOS and HA tag expression levels are progressively higher as the plasmid dose increases. GAPDH was used as a loading control.

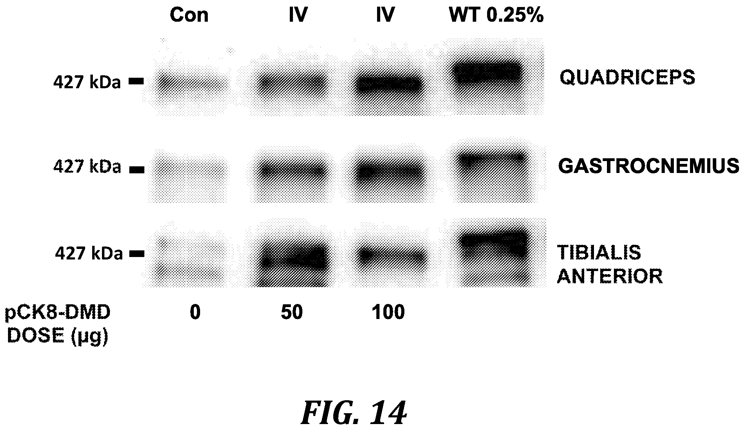

FIGURE 14 illustrates results from Experiment 9, where expression of full-length dystrophin in muscles was measured following systemic delivery of the non-viral platform composition (see Fig. 8). Two *mdx*4CV mice were injected I.V. with different doses of the platform containing 50 or 100 µg of the CK8-dystrophin construct. As shown by Western blot, both doses show full-length dystrophin expression in three hindlimb muscles (Quadriceps, Gastrocnemius and Tibialis Anterior), with levels ~0.2 to .25% of a WT mouse. The total protein loaded into the WT well was the same as all other wells, but consisted of 0.25% WT protein mixed with 99.75% *mdx*4CV Con protein. The faint bands in the non-injected *mdx*4CV Control mouse are indicative of the very small number of revertant fibers that express low levels of dystrophin in *mdx* mice.

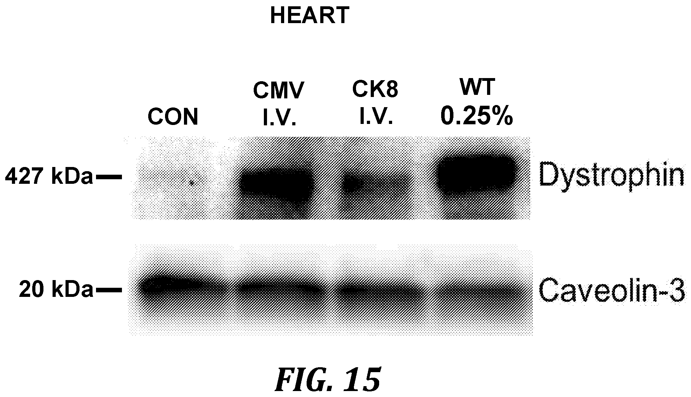

FIGURE 15 highlights results from Experiment 9 showing expression of full-length dystrophin in cardiac muscle following systemic delivery of the platform composition (see Fig. 8). Two *mdx*4CV mice were injected I.V. with the platform containing either CK8-dystrophin or the CMV-dystrophin construct. As shown by Western blot, both constructs resulted in full-length dystrophin expression in the Heart with the CMV-dystrophin plasmid displaying a higher level than the CK8 construct. Here, the expression level in cardiac muscle was also ~0.1-0.2% of a WT mouse. The total protein loaded into the WT well was the same as all other wells but consisted of 0.25% WT protein mixed with 99.75% *mdx*4CV Con protein. Caveolin-3 was used as a membrane-specific loading control.

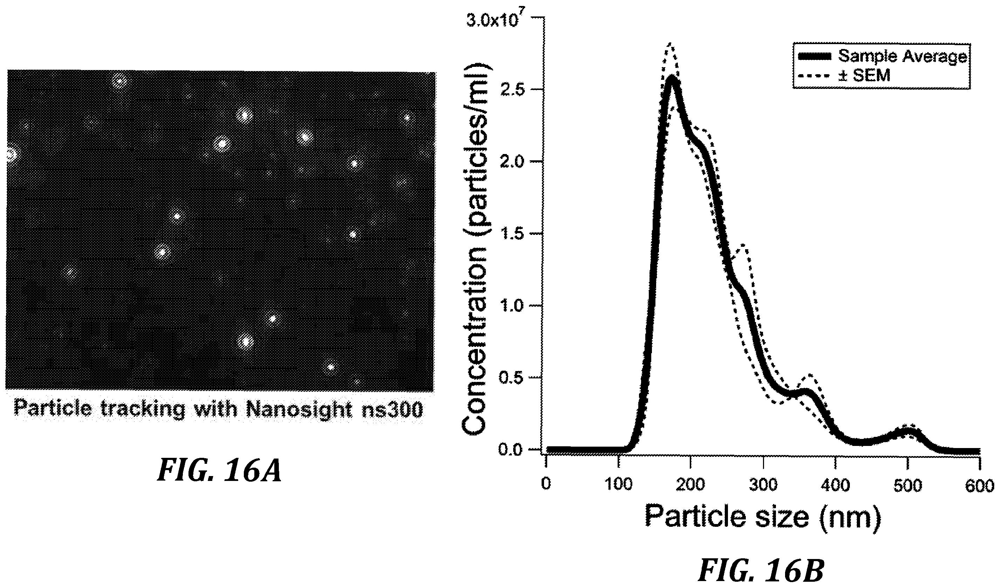

FIGURE 16A-B demonstrates results from Experiment 10, showing particle tracking analysis of the non-viral platform composition (see Fig. 8) containing the CK8-full-length dystrophin plasmid. (16A) An image of the platform particles identified and tracked by the Nanosight ns300 software for size analysis. (16B) A plot of the particle distribution, showing the particle sizes against particle concentration. Values are the mean ± SEM of 4 separate inputs from the same sample.

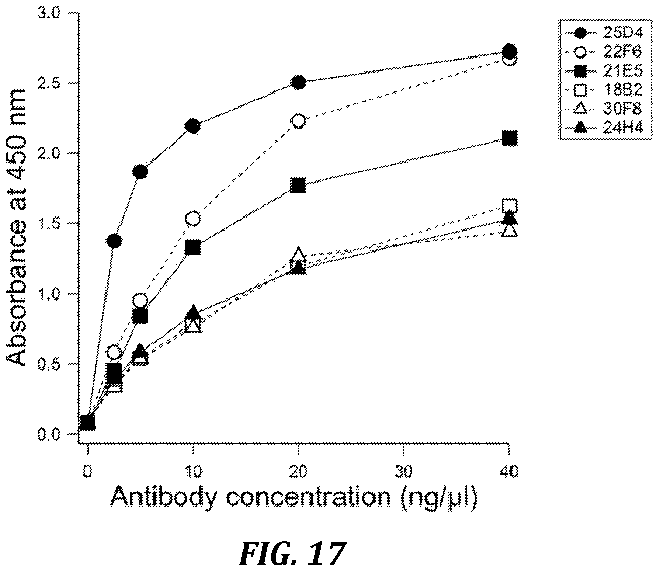

FIGURE 17 illustrates results from Experiment 11, where an ELISA assay was designed to measure the binding affinity of 6 monoclonal antibodies made against the mouse peptide sequence of GLUT4 (GRQGPGGPDSI; SEQ ID NO:4). As shown, all antibodies displayed a dose-dependent increase in absorbance, indicative of increased binding to the mouse GLUT4 peptide.

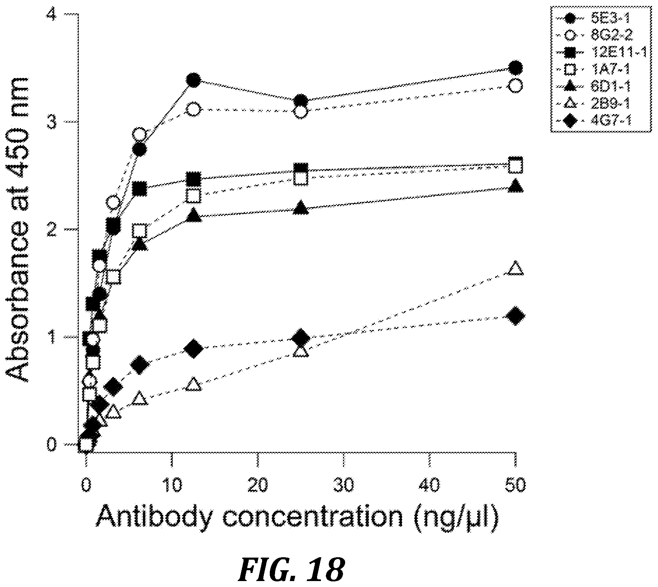

FIGURE 18 demonstrates results from Experiment 11 of an ELISA assay aimed at testing the binding affinity of 7 monoclonal antibodies made against the human peptide sequence of GLUT4 (GRQGPEGPSSI; SEQ ID NO:2.). As presented, all antibodies displayed a dose-dependent increase in absorbance, indicative of increased binding to the recombinant human GLUT4 protein.

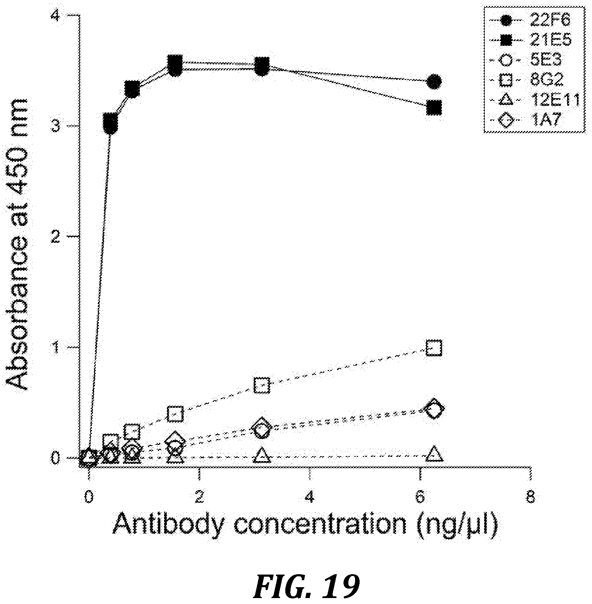

FIGURE 19 shows the results from Experiment 11, where an ELISA assay was designed to test whether antibodies made against the human GLUT4 sequence (GRQGPEGPSSI; SEQ ID NO:2.) could bind to the mouse GLUT4 peptide sequence (GRQGPGGPDSI; SEQ ID NO:4). As displayed, antibody clones made against the human sequence (5E3, 8G2, 12E11, 1A7) bound the mouse peptide much more weakly than antibodies made against the mouse sequence (22F6 and 21E5), which reached binding saturation at a relatively low concentration (~2 ng/ml).

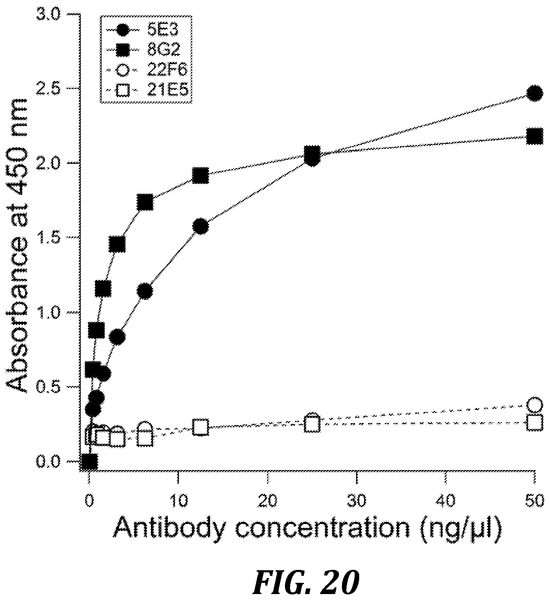

FIGURE 20 demonstrates the ELISA assay results from Experiment 11, aimed at testing whether antibodies made against the mouse GLUT4 sequence (GRQGPGGPDSI; SEQ ID NO:4) could bind to the human GLUT4 sequence (GRQGPEGPSSI; SEQ ID NO:2.). As shown, antibodies made against the mouse sequence (22F6 and 21E5) bound very weakly to the antigen, while antibodies to the human sequence bound strongly (5E3, 8G2).

### DETAILED DESCRIPTION

This disclosure is based on the inventors' development of non-viral vector platforms for specifically targeting therapeutic payloads, both genetic and pharmacological, to skeletal and cardiac muscle tissue. Briefly, the platform design, outlined in Fig. 2, was focused on permitting large payload capacities, such as the full-length dystrophin gene transcript to permit treatment of diseases relating to dysfunction of the dystrophin expression or gene product.

The platform leverages the specific and insulin-dependent expression of glucose transporter 4 (GLUT4) protein in muscle and other limited tissues. GLUT4 is encoded by the SLC2A4 gene, which is predominantly expressed in striated muscle (skeletal and cardiac) and adipose tissue, but is also expressed in certain areas of the brain, including the hippocampus and cerebellum. GLUT4 is a member of a family of glucose transport proteins. Glucose transporters are integral membrane proteins containing 12 membrane-spanning helix domains. GLUT4 is highly expressed in muscle and fat tissue while other members of the glucose transporter family are specific to other tissues. For example, GLUT3, a high-affinity glucose transporter, is the primary glucose transporter in neurons. Under basal conditions, GLUT4 mostly resides in intracellular vesicles but traffics to, and fuses with, the plasma membrane upon treatment with insulin, thereby mediating insulin-dependent glucose uptake. Following insulin binding to the insulin receptor, a signaling pathway is activated, leading to GLUT4 translocation to the plasma membrane. This process occurs within 15 min of insulin binding. Once insulin concentrations decrease, over time GLUT4 is internalized in vesicles and either traffics through the endosomal system or is recycled back to the plasma membrane.

The disclosed vector platform leverages this bidirectional, vesicle-mediated movement of GLUT4 to the plasma membrane and then back inside the cell to provide a strategy to deliver therapeutics into GLUT4 expressing cells and tissues. As described in more detail below, novel antibodies, both rabbit polyclonal and mouse monoclonal, were first developed that bind to an extracellular domain of the mouse GLUT4 protein. Using various antibody conjugation techniques, several payload/vector structures were produced. A series of *in vitro* and *in vivo* experiments were conducted to assess the specificity, carrying capacity, and other useful aspects of these representative embodiments of the platform vector. For example, it was demonstrated that an effectively targeted therapeutic transgene, including plasmid DNA as large as 19 kilobases containing the full-length dystrophin gene, can be taken up and expressed by muscle cells. The plasmid DNA was successfully transported into the cell following GLUT4 internalization and expression of the dystrophin gene was established. The assays ultimately demonstrate the usefulness of the platform for addressing muscle diseases that have heretofore been difficult to treat. Not only is the platform flexible to permit a variety of therapeutic payload types, it also does not require viral components. Accordingly, the platform permits repeated administration over time, including periods of years, to address chronic and degenerative muscle diseases without risk of immunogenicity.

In accordance with the foregoing, the disclosure provides a composition comprising a binding domain that specifically binds to glucose transporter 4 ("GLUT4") protein. The composition further comprises a therapeutic payload conjugated to the binding domain.

### Binding domain

In some embodiments, the binding domain is or comprises an antibody, or fragment or derivative thereof. In such embodiments, the therapeutic payload can be directly or indirectly conjugated to antibody, or fragment or derivative thereof.

The term "antibody" is used herein in the broadest sense and encompasses various antibody structures derived from any antibody-producing mammal (e.g., mouse, rat, rabbit, and primate including human), and which specifically bind to an antigen of interest. An antibody fragment specifically refers to an intact portion or subdomain of a source antibody that still retains antigen-binding capability. An antibody derivative refers to a molecule that incorporates one or more antibodies or antibody fragments. Typically, there is at least some additional modification in the structure of the antibody or fragment thereof, or in the presentation or configuration of the antibody or fragment thereof. Exemplary antibodies of the disclosure include polyclonal, monoclonal and recombinant antibodies. Exemplary antibodies or antibody derivatives of the disclosure also include multispecific antibodies (e.g., bispecific antibodies); humanized antibodies; murine antibodies; chimeric, mouse-human, mouse-primate, primate-

As indicated, an antibody fragment is a portion or subdomain derived from or related to a full-length antibody, preferably including the complementarity-determining regions (CDRs), antigen binding regions, or variable regions thereof, and antibody derivatives refer to further structural modification or combinations in the resulting molecule. Illustrative examples of antibody fragments or derivatives encompassed by the present disclosure include Fab, Fab', F(ab)₂, F(ab')₂ and Fv fragments, diabodies, single-chain antibody molecules, V_{H}H fragments, V_{NAR} fragments, multispecific antibodies formed from antibody fragments, nanobodies and the like. For example, an exemplary single chain antibody derivative encompassed by the disclosure is a "single-chain Fv" or "scFv" antibody fragment, which comprises the V_{H} and V_{L} domains of an antibody, wherein these domains are present in a single polypeptide chain. The Fv polypeptide can further comprise a polypeptide linker between the V_{H} and V_{L} domains, which enables the scFv to form the desired structure for antigen binding. Another exemplary single-chain antibody encompassed by the disclosure is a single-chain Fab fragment (scFab).

As indicated, antibodies can be further modified to created derivatives that suit various uses. For example, a "chimeric antibody" is a recombinant protein that contains domains from different sources. For example, the variable domains and complementarity-determining regions (CDRs) can be derived from a non-human species *(e.g.,* rodent) antibody, while the remainder of the antibody molecule is derived from a human antibody. A "humanized antibody" is a chimeric antibody that comprises a minimal sequence that conforms to specific complementarity-determining regions derived from non-human immunoglobulin that is transplanted into a human antibody framework. Humanized antibodies are typically recombinant proteins in which only the antibody complementarity-determining regions (CDRs) are of non-human origin. Any of these antibodies, or fragments or derivatives thereof, are encompassed by the disclosure.

Antibody fragments and derivatives that recognize specific epitopes can be generated by any technique known to those of skill in the art. For example, Fab and F(ab')₂ fragments of the disclosure can be produced by proteolytic cleavage of immunoglobulin molecules, using enzymes such as papain (to produce Fab fragments) or pepsin (to produce F(ab')₂ fragments). F(ab')₂ fragments contain the variable region, the light chain constant region and the CHI domain of the heavy chain. Further, the antibodies, or fragments or derivatives thereof, of the present disclosure can also be generated using various phage display methods known in the art. Finally, the antibodies, or fragments or derivatives thereof, can be produced recombinantly according to known techniques.

It will be apparent to the skilled practitioner that the binding domain can comprise antigen binding molecules other than antibody-based domain, such as peptidobodies, antigen-binding scaffolds (e.g., DARPins, HEAT repeat proteins, ARM repeat proteins, tetratricopeptide repeat proteins, and other scaffolds based on naturally occurring repeat proteins, etc. [see, *e.g.,* Boersma and Pluckthun, Curr. Opin. Biotechnol. 22:849-857, 2011, and references cited therein, incorporated herein by reference]), which include a functional binding domain or antigen-binding fragment thereof.

In some embodiments, the binding domain *(e.g.,* an antibody, or fragment or derivative thereof) is biotinylated. As described in more detail below, biotinylation facilitates some embodiments of composition assembly/conjugation.

As used herein, the term "specifically bind" or variations thereof refer to the ability of the binding domain *(e.g.,* of the antibody, or fragment or derivative thereof) to bind to the antigen of interest (e.g., GLUT4), without significant binding to other molecules, under standard conditions known in the art. The binding domain can bind to other peptides, polypeptides, or proteins, but with lower affinity as determined by, e.g., immunoassays, BIAcore, or other assays known in the art. However, the binding domain preferably does not substantially cross-react with other antigens.

In some embodiments, the GLUT4-binding domain of the composition has a binding affinity has a binding affinity within a range characterized by a dissociation constant (*K*_{d}) from about 50nM (lower binding affinity) to about 0.001 nM (higher binding affinity). For example, the binding domain has a binding affinity for the GLUT protein characterized by (*K*_{d}) of about 50nM, 40nM, 30nM, 20nM, 10nM, 5nM, 1nM, 0.75nM, 0.5nM, 0.1nM, 0.05nM, 0.01nM, 0.005nM, and 0.001nM, or even smaller. Typical (*K*_{d}) ranges characterizing the binding affinity of the cell-targeting domain for the antigen characteristic of the cell-type of interest include from about 30nM to about 10nM, from about 20nM to about 1nM, from about 10nM to about 0.1nM, from about 0.5nM to about 0.05nM, and from about 0.1nM to about .001nM, or even lower, or any subrange therein.

### Binding target

As indicated above, the binding domain (e.g., an antibody, or fragment or derivative thereof) specifically binds to glucose transporter 4 (GLUT4) protein. GLUT 4 is encoded by the SLC2A4 gene (or homologs thereof), which is predominantly expressed in striated muscle (skeletal and cardiac) and adipose tissue, but is also expressed in certain areas of the brain, including the hippocampus and cerebellum. This disclosure encompasses GLUT4 from any mammal, such as human, mouse, rat, cat, dog, horse, etc. In some embodiments, the binding domain specifically binds to an extracellular domain of GLUT4 protein.

In some embodiments, the binding domain specifically binds to a first extracellular loop domain of GLUT4. In some embodiments, the first extracellular loop domain of GLUT4 comprises an amino acid sequence with at least about 80% sequence identity *(e.g.,* at least about 80%, at least about 85%, at least about 90%, at least about 92%, at least about 94%, at least about 96%, at least about 98%, and 100% sequence identity) to the sequence set forth in SEQ ID NO:2, which is a subdomain of a human GLUT4 first extracellular loop domain. For example, exemplary GLUT4 domain contemplated by the present disclosure include the domains set forth in Genbank Accession No. M91463.1 (human), Genbank Accession No. AB0084531 (mouse), Genbank Accession No. NM_001159327.1 (dog), and Genbank Accession No. L36125.1 (rat), each of this is incorporated herein by reference in its entirety. In some embodiments, the GLUT4 first extracellular loop domain comprises an amino acid sequence with at least about 80% sequence identity (e.g., at least about 80%, at least about 85%, at least about 90%, at least about 92%, at least about 94%, at least about 96%, at least about 98%, and 100% sequence identity) to the sequence set forth in SEQ ID NO:1, which is a representative human GLUT4 first extracellular loop domain. In some embodiments, the binding domain specifically binds to a subdomain of the first extracellular loop domain of GLUT4 that comprises a sequence with at least 80% sequence identity (e.g., at least about 80%, at least about 85%, at least about 90%, at least about 92%, at least about 94%, at least about 96%, at least about 98% sequence identity, and 100% sequence identity) to the sequence set forth in SEQ ID NO:2.

In some embodiments, the first extracellular loop domain of GLUT4 comprises an amino acid sequence with at least about 80% sequence identity *(e.g.,* at least about 80%, at least about 85%, at least about 90%, at least about 92%, at least about 94%, at least about 96%, at least about 98%, and 100% sequence identity) to the sequence set forth in SEQ ID NO:4, which is a subdomain of a murine GLUT4 first extracellular loop domain. In some embodiments, the GLUT4 first extracellular loop domain comprises an amino acid sequence with at least about 80% sequence identity (e.g., at least about 80%, at least about 85%, at least about 90%, at least about 92%, at least about 94%, at least about 96%, at least about 98%, and 100% sequence identity) to the sequence set forth in SEQ ID NO:3, which is a representative murine GLUT4 first extracellular loop domain. In some embodiments, the binding domain specifically binds to a subdomain of the first extracellular loop domain of GLUT4 that comprises a sequence with at least 80% sequence identity (e.g., at least about 80%, at least about 85%, at least about 90%, at least about 92%, at least about 94%, at least about 96%, at least about 98% sequence identity) to the sequence set forth in SEQ ID NO:4.

### Payload

The therapeutic payload can be any payload capable of inducing a change in a target cell that expresses, at least transiently, a GLUT4 on its surface. The change can be to a therapeutic effect if it is *in vivo.* Exemplary, non-limiting therapeutic payloads can comprise a nucleic acid, a protein or peptide, a lipid, a small molecule pharmaceutical, and/or a radioisotope.

For example, in some embodiments, the therapeutic payload comprises a nucleic acid, such as DNA (including cDNA), mRNA, siRNA, shRNA, and gRNA. Guide RNA molecules can be utilized in applications of DNA editing (e.g., CRISPR/Cas9 applications) and RNA editing.

In some embodiments, the nucleic acid comprises a sequence *(e.g.,* open reading frame) encoding a protein that has a therapeutic effect in the target cell. In some embodiments, the nucleic acid comprises an open reading frame operatively linked to a promoter sequence to provide for expression of the open reading frame in the target cell. This open reading frame can be referred to as a transgene. The term "promoter" refers to a regulatory nucleotide sequence that can activate transcription (expression) of the transgene and/or splice variant isoforms thereof. The promoter is typically located upstream of the gene, but can be located at other regions proximal to the gene, or even within the gene. The promoter typically contains binding sites for RNA polymerase and one or more transcription factors, which participate in the assembly of the transcriptional complex. As used herein, the term "operatively linked" indicates that the promoter and the encoding nucleic acid are configured and positioned relative to each other a manner such that the promoter can activate transcription of the encoding nucleic acid by the transcriptional machinery of the cell. The promoter can be constitutive or inducible. Constitutive promoters can be determined based on the character of the target cell and the particular transcription factors available in the cytosol. A person of ordinary skill in the art can select an appropriate promoter based on the intended person, as various promoters are known and commonly used in the art Exemplary, non-limiting promoters include muscle creatine kinase (CK) promoter (Genbank Accession No. AF188002.1) and human skeletal actin promoter (Genbank Accession No. NG_006672.1), each of which is incorporated herein by reference. While the advances provided herein permit use of full-length promoters, abbreviated promoters that retain some activity are still encompassed by the present disclosure. Exemplary, non-limiting examples of abbreviated CK promoters that retain activity are described in Hauser MA, et al., Analysis of muscle creatine kinase regulatory elements in recombinant adenoviral vectors, Mol Ther. 20001 2(1):16-25, and Brennan KJ and Hardeman EC, Quantitative analysis of the human alpha-skeletal actin gene in transgenic mice, J Biol Chem. 1993 268(1):719-725, each of which is incorporated herein by reference in its entirety.

The nucleic acid therapeutic payload can be of any size and in any configuration, such as in linear form, in plasmid form (e.g., circular form), or in minicircle form. As demonstrated below, the compositions of the present disclosure were demonstrated as being able to deliver very large transgenes for successful expression in target cells. Thus, the nucleic acid payloads are not limited in a manner that practically restricts the use of typical viral vectors. Thus, linear forms of nucleic acids can encompass short oligos or longer transgenes, without limitation. As understood by skilled practitioners, minicircles are smaller circular plasmid derivatives that lack most or all prokaryotic vector components. Minicircles can function as transgene carriers with an advantage of being less susceptible to degradation due to the smaller size and/or the lack of prokaryotic sequence.

As indicated above, the nucleic acid can be a transgene configured to comprise a sequence encoding any desired protein or protein fragment to be expressed in the target cell. The transgene can encode any protein that is desired to be expressed in the cell. In some embodiments, the transgene encodes a protein that has a beneficial effect on the cell compared to a mutant endogenous version existing in the target cell. For example, as described above, Duchenne muscular dystrophy (DMD) is a debilitating sex-linked disease resulting from aberrant dystrophin protein (or aberrant dystrophin protein expression). The gene encoding dystrophin, referred to as the "DMD" gene, is one of the longest human genes, covering about 2.3 megabases (0.08% of the human genome) at locus Xp21 with about 79 exons. The primary wild-type transcript is about 21 kilobases, and the mature mRNA is about 14 kilobases. As described below, the inventors established that the disclosed vector platform can deliver such long transgenes to GLUT4+ cells *(e.g.,* muscle cells) to successfully achieve expression. Accordingly, an exemplary and non-limiting example of a nucleic acid payload encompasses the encoding nucleotide sequence encoding dystrophin protein, or a functional fragment thereof, operatively linked to a promoter sequence. In some embodiments, the encoded dystrophin protein is a full length human dystrophin protein or a substantially full length human dystrophin protein. An exemplary full length protein sequence for human dystrophin is set forth in SEQ ID NO:5. In some embodiments, the nucleic acid payload encodes a dystrophin with at least about 80% sequence identity (e.g., at least about 80%, at least about 85%, at least about 90%, at least about 92%, at least about 94%, at least about 96%, at least about 98%, and 100% sequence identity) to the sequence set forth in SEQ ID NO:5.

The term "substantially full length" encompasses embodiments where a significant but incomplete version of dystrophin is encoded, such as at least about 85%, at least about 90%, at least about 92%, at least about 94%, at least about 96%, at least about 98%, or at least about 99% of a full length dystrophin protein (e.g., with at least 80% sequence identity to SEQ ID NO:5, as described above) is encoded providing a degree of phenotypic improvement in the target cell.

It has also been shown that "micro-dystrophin" proteins administered to or expressed in a cell with an aberrant or mutated DMD gene can provide at least some ameliorative effect compensating for the endogenous mutation in the DMD gene. While not being bound to any particular theory, it is believed that many truncated versions of dystrophin have improved functionality over the mutated or aberrant expression of dystrophin in subjects with DMD and other dystrophin related diseases. Accordingly, delivery or inducement of expression of these truncated or micro-dystrophin proteins with the muscle cell can begin to restore a relatively healthy or improved phenotype. Accordingly, in some embodiments, the nucleic acid payload can encode a functional fragment of the dystrophin protein, such as any micro-dystrophin known in the art. For example, Ramos et al., 2019 Molecular Therapies 27: 623-635, incorporated herein by reference, describe several miniaturized micro-dystrophin constructs that are optimized to improve performance. Exemplary and non-limiting regions and domains of the dystrophin protein that have been shown to possess characteristics contributing to functionality of micro-dystrophin constructs include: a rod domain with at least 4 SR domains, α-syntrophin-binding domain in SR16-17, an actin binding domain, and cysteine-rich domain for binding β-dystroglycan.

In some embodiments, the full length dystrophin protein has an amino acid sequence with at least 80% sequence identity (e.g., at least about 80%, at least about 85%, at least about 90%, at least about 92%, at least about 94%, at least about 96%, at least about 98%, and 100% sequence identity) to the sequence set forth as SEQ ID NO:5, which is a predicted full-length human protein sequence for dystrophin. The sequence has a Genbank accession number AAA53189.1 and is incorporated herein by reference in its entirety. In other embodiments, the full length dystrophin protein has an amino acid sequence with at least 80% sequence identity (*e.g.*, at least about 80%, at least about 85%, at least about 90%, at least about 92%, at least about 94%, at least about 96%, at least about 98%, and 100% sequence identity) to the sequence set forth as SEQ ID NO:6, which is a predicted full-length murine protein sequence for dystrophin. The sequence has a Genbank accession number NP_031894.1 and is incorporated herein by reference in its entirety. In yet other embodiments, the full length human dystrophin protein has an amino acid sequence with at least 80% sequence identity *(e.g.,* at least about 80%, at least about 85%, at least about 90%, at least about 92%, at least about 94%, at least about 96%, at least about 98%, and 100% sequence identity) to the sequence set forth as SEQ ID NO:7, which is a predicted full-length rat protein sequence for dystrophin. The sequence has a Genbank accession number XP_017457396.1 and is incorporated herein by reference in its entirety. In yet other embodiments, the full length human dystrophin protein has an amino acid sequence with at least 80% sequence identity *(e.g.,* at least about 80%, at least about 85%, at least about 90%, at least about 92%, at least about 94%, at least about 96%, at least about 98%, and 100% sequence identity) to the sequence set forth as SEQ ID NO:8, which is a predicted full-length rabbit protein sequence for dystrophin. The sequence has a Genbank accession number XP_017205220.1 and is incorporated herein by reference in its entirety. As indicated above, the nucleic acid therapeutic payload in these embodiments can comprise the full length dystrophin protein sequence, substantially full length dystrophin sequence, or be a functional fragment thereof that retains the ability to provide at least some therapeutic effect towards restoring an improved phenotype.

As indicated above, the nucleic acid payload can comprise linear nucleic acids, such short oligomers. In some embodiments, the nucleic acid is not a transgene that encodes a protein, but rather alters the endogenous expression and translation of proteins in the target cell. For example, in some therapeutic approaches for treating genomic diseases such as DMD, nucleic acid oligos are administered to promote skipping of exons in the pre-mRNA that contain a mutation resulting in a frameshift. The administered nucleic acid oligos (*e.g.,* phosphodiamedate morpholino oligomers) are configured to hybridize to the mutated pre-mRNA in a sequence specific manner and sterically hinder spliceosome from binding at the junctions of the mutated exon in the encoding pre-mRNA molecule. The result is that the spliceosome excises not only the introns but the exon containing the frameshifting mutation. The resulting mature mRNA encodes for a slightly truncated protein lacking the mutated exon and maintaining the reading frame along all the remaining introns. The truncated protein retains at least some wild-type characteristics compared to the version containing the frameshift. This approach has been implemented for DMD. However, typical approaches require very large quantities of oligos due to the high rate of degradation after administration. See, e.g., Verhaart IEC and Aartsma-Rus A, Therapeutic developments for Duchenne muscular dystrophy, Nat Rev Neurol. 2019 Jul;15(7):373-386, incorporated herein by reference in its entirety. The nucleic acid payload of the present disclosure encompasses therapeutic oligomers such as these. The disclosed platform provides the advantage of specific targeting of the oligomers to the target cell, which leads to efficient uptake into the cell. Thus, the disclosed targeting platform does not require such high doses of oligos and provides higher likelihood of delivery to and uptake by the target cells.

In other embodiments, the nucleic acid payload of the present disclosure can be configured to promote RNA interference. For instance, in some embodiments the nucleic acid payload is or comprises short interfering RNA (siRNA) or short hairpin RNA (shRNA) molecules, or nucleic acid encoding the siRNA or shRNA molecules. The sequences of such nucleic acid molecules can be readily configured according to skill and knowledge in the art to result in binding to and subsequence degradation of mRNA transcripts to result in knockdown of any desired target protein in the cell.

In any embodiment of a nucleic acid payload, but particularly when the nucleic acid payload is quite large (for example for long nucleic acids encoding dystrophin or large portions thereof) the composition can further comprise additional components to facilitate efficient coiling or packaging (e.g., condensing) of the nucleic acid. The additional packaging can be useful for linear *(e.g.,* oligo or long sequences), plasmid, and minicircle embodiments of the nucleic acid payload, as described above, without limitation. For example, in one embodiment, the composition further comprises a histone protein or a plurality of histone proteins associated with the nucleic acid. The histone protein(s) can facilitate the coiling of the nucleic acid payload for effective packaging and transfer. In some embodiments, the histone protein or at least a portion of the plurality of histone proteins is/are biotinylated to facilitate conjugation, as described in more detail below.

In other embodiments, the composition further comprises a plurality of cationic moieties associated with the nucleic acid, thereby resulting in condensed nucleic acid. Without being limited to any particular theory, cationic molecules can potentially condense DNA, which is a polymeric anionic molecule. Thus, peptides containing a plurality of positively charged amino acids are useful for condensing the polymeric nucleic acid payload. Exemplary cationic amino acids include Lysine (Lys, K), Histidine (His, H), and Arginine (Arg, R). Accordingly, in some embodiments, the composition further comprises one or more cationic peptides, where the cationic peptides comprise a plurality of cationic amino acids, *e.g.,* selected from His, Arg, and Lys, potentially in combination. The peptide typically has a net positive charge. In some embodiment, the peptide comprises one or more poly-L-Lys ("PLL") peptide moieties. Additional, non-limiting cationic molecules or moieties that can be included in the composition to condense the nucleic acid payload include linear Polyethylenimine ("LPEI"), branched Polyethylenimine ("BPEI"), chitosan, spermidine and spermine, Polyamidoamine ("PAMAM"), poly(2-dimethylaminoethyl methacrylate) ("PDMAEMA"), Poly(beta-amino ester)s ("PBAEs"), poly{N-[N-(2-aminoethyl)-2-aminoethyl]aspartamide ("PAsp(DET)"), poly(2-aminoethyl ethylene phosphate ("PPEEA"), and the like.

In some embodiments, at least a portion of the cationic moieties, such as PLL or other cationic molecules indicated above, is biotinylated to facilitate conjugation, as described in more detail below.

### Conjugation

The binding domain, e.g., antibody, or fragments or derivative thereof, can be conjugated to the therapeutic payload according to any known technique. The conjugation can be covalent or ionic.

In some illustrative, non-limiting embodiments, the conjugation relies on the interaction between biotin and a binding partner, such as avidin, neutravidin, and streptavidin, to provide a very high affinity and specific conjugation of the component parts of the composition.

For example, in one embodiment, the binding domain (e.g., antibody, or fragment or derivative thereof), and a DNA coiling or condensing element (e.g., histone or cationic peptide) are each biotinylated. The nucleic acid payload is associated with the DNA coiling or condensing element (e.g., coiled around or condensed by the coiling or condensing element). Through the mutual interactions with the multiple biotinylated components with the same neutravidin molecule, the binding domain and the coiling or condensing element and associate nucleic acid payload are conjugated together with high affinity.

In some further embodiments, a plurality of binding domains and nucleic acid payloads (with associated DNA coiling or condensing elements) can be aggregated. This can be accomplished simply by having multiple biotinylated elements bound to the same avidin-based binding partner. Alternatively, there can be multiple biotinylated DNA coiling or condensing elements associated with the same nucleic acid payload, each of which is bound to a different avidin-based binding partner, which in turn is bound to a different binding domain. This aggregation can be further promoted by inclusion in the composition of one or more particles, such as liposomes, which can associate with multiple nucleic acid payloads. For example, as illustrated in FIGURE 3, multiple nucleic acid plasmid payloads are associated with a cationic liposome structure. Each plasmid is in turn associated with a plurality of histone proteins, a portion of which are biotinylated. The biotinylated histones are bound to biotinylated binding domains (e.g., anti-GLUT4 antibodies) by mutual binding to neutravidin. The generation of such exemplary composition is described in more detail below.

Accordingly, in some embodiments, the disclosed composition can further comprise a cationic particle, such as a liposome, that can further associate and/or aggregate with the nucleic acid payload. Cationic liposomes are known and can be readily constructed by persons of ordinary skill in the art to facilitate proper aggregation and/or association with the nucleic acid payload. In some illustrative, non-limiting embodiments the liposome can comprise one or more lipids selected from DC-Cholesterol·HCl,3b-[N-(N',N'-dimethylaminoethane)-caramoyl]cholesterol hydrochloride ("DC-Chol"), 1,2-dioleoyl-sn-glycero-3-phosphoethanolamine ("DOPE"), 1,2-di-O-octadecenyl-3-trimethylammonium ("DOTMA"), N-[1-(2,3-Dioleoyloxy)propyl]-N,N,N-trimethylammonium propane ("DOTAP"), dimethyldioctadecylammonium bromide ("DDAB"), 1,2-dimyristoyl-3-trimethylammonium-propane ("DMTAP"), 1,2-dioleoyl-3-dimethylammonium-propane ("DODAP"), cholesteryl hemisuccinate ("CHEMS") and cholesterol ("CHOL"), 2,3-dioleyloxy-N-[2(sperminecarboxamido)ethyl]-N,N-dimethyl-1-propanaminium ("DOSPA"), Di-octadecyl-amido-glycyl-spermine ("DOGS"), dioleoylphosphatidylcholine ("DOPC"), and the like. In some embodiments, the liposome further comprises polyethylene glycol ("PEG").

In another exemplary embodiment, illustrated in FIGURE 8, a plasmid DNA payload is associated with a plurality of PLL cationic moieties, which are biotinylated. The biotinylated PLL moieties are bound to the binding domains (e.g., anti-GLUT4 antibodies) by mutual association with the same neutravidin binding partner.

In additional embodiments, the composition further comprises albumin, which can contribute to the enhanced expression of the nucleic acid payloads. As described in more detail below, recent evidence has shown that albumin can enhance protein expression from delivered DNA/liposomal vectors. Among other reasons, albumin is believed to enhance delivery of the composition to the target cells, including enhancing endosomal escape and nuclear uptake of the nucleic acid payload. Albumin, which is negatively charged, can bind to positively charged liposomes that are associated with the nucleic acid payloads. In other embodiments where biotin and avidin-based binding partners are used for conjugation, the albumin can be biotinylated. Thus, a positively charged liposome is not a prerequisite for efficacy of albumin. This is illustrated in FIGURE 8.

In one aspect, the disclosure provides the compositions described herein for use in treating a disease, wherein the disease is treatable by delivery of a payload to a cell that expresses GLUT4. Applicable diseases encompassed by this aspect, in addition to other elements of "treating" as used herein, are described in more detail below. In some embodiments, the use is for treating a disease or condition in a subject with a genetic mutation in a gene encoding dystrophin protein. In some embodiments, the compositions described herein are for use in treating of Duchenne muscular dystrophy ("DMD") or Becker muscular dystrophy ("BMD"), which are described in more detail above and below. In these embodiments, the payload of the disclosed compositions can comprise a nucleic acid that encodes full-length (e.g., wildtype) dystrophin protein, or a functional fragment thereof (e.g., about 80%, 85%, 90%, 95%, 98%), or a variant thereof with at least about 90%, 95%, or 98% amino acid sequence identity to a wildtype sequence. In some embodiments, variation in sequence identity from a wildtype sequence results from conservative substitutions that do not significantly affect protein function. In some embodiments, the use can further comprise administering to the subject insulin to increase the accessible GLUT4 target and targeting of the payload.

### Methods

The disclosure also encompasses methods of using and making the disclosed compositions.

In one aspect, the disclosure provides a method of targeting a payload (e.g., therapeutic payload) for delivery to a cell that expresses GLUT4. The method comprises contacting the cell with any embodiment of the disclosed composition, e.g., as recited above.

In some embodiments, the method further comprises contacting the cell with insulin prior to contacting the cell with the composition. As described above, GLUT4 is highly expressed in striated muscle and fat tissue, in addition in cells within certain areas of the brain, including the hippocampus and cerebellum. GLUT4 mostly resides in intracellular GLUT4 storage vesicles (GSV). However, following insulin binding to the insulin receptor (in insulin-sensitive cells including skeletal muscle, heart and fat cells and some neurons), a signaling pathway is activated, leading to GLUT4 translocation to the plasma membrane. See, e.g., Klip, A, et al., 30 sweet years of GLUT4. 2019, REV119.008351, incorporated herein by reference in its entirety. Thus, in some embodiments, insulin is contacted to the cell at least about 10 minutes (such as about 10, 15, 20, 25, 30, 35, 40, 45, 50, 55, 60, 80, 90, 120 minutes or more) prior to contacting the cell with the composition.

In some embodiments, the cell is a skeletal muscle cell, a cardiac muscle cell, a smooth muscle cell, an adipose tissue cell, a hippocampal cell, or a cerebellum cell.

As indicated above, the payload (e.g., therapeutic payload) can be any composition that improves or alters the phenotype of the target cell. In some embodiments, the therapeutic payload is a nucleic acid, such as a transgene that provides for expression of a desired protein in the cell. An illustrative embodiment is a transgene encoding dystrophin, or a functional fragment thereof, to provide for expression of a dystrophin protein in the cell. Accordingly, in some embodiments, the method is characterized as a method of expressing, or increasing expression of, functional dystrophin protein in the cell, or increasing expression of, a functional fragment of dystrophin protein in the cell. The increase can be any expression level from a starting point of no expression prior to performance of the method. In some embodiments, the cell can have residual (i.e., detectable) expression of the dystrophin protein or a functional fragment thereof. In some embodiments, the cell can express dystrophin protein or a functional fragment thereof at a level that is at least about 0.01%, 0.025%, 0.05%, 0.1%, 1%, 2.5%, 5%, 10%, 25%, 20%, 25%, 30%, 35%, 40%, 45%, 50%, 55%, 60%, 65%, 70%, 75%, 80%, 85%, 90%, 95%, 100%, or more as compared to a similar cell (e.g., from a same tissue source) that has an intact wild-type gene encoding dystrophin. In some embodiments, the cell is a muscle cell.

In other embodiments, the cell is a cancer cell that expressed GLUT4 on the surface. Non-limiting examples of such cancer cells include melanoma cells, prostate cancer cells, cancer cells of muscle tissue, such as rhabdomyosarcoma cell, and breast cancer cells. In such embodiments, the therapeutic payload can be any anti-cancer therapeutic payload, such as a radioisotope or other toxin.

The method can be performed *in vitro.* Alternatively, the method can be an *in vivo* method directed to treatment of a disease associated with dysfunction of cells that express GLUT4, either constitutively or transiently, on the surface. The *in vivo* method comprises administering an effective amount of the disclosed composition to the subject in need thereof.

As used herein, the term "treat" refers to medical management of a disease, disorder, or condition (e.g., muscular dystrophy, such as Duchenne muscular dystrophy (DMD) or Becker muscular dystrophy (BMD) or cancer) of a subject (e.g., a human or non-human mammal, such as another primate, horse, dog, mouse, rat, guinea pig, rabbit, and the like). Treatment can encompass any indicia of success in the treatment or amelioration of a disease or condition (e.g., DMD, BMD, or cancer), including any parameter such as abatement, remission, diminishing of symptoms or making the disease or condition more tolerable to the patient, slowing in the rate of degeneration or decline, or making the degeneration less debilitating. In non-limiting examples, the term "treat" in the context of DMD can encompass slowing or inhibiting the progression of muscle loss or weakness; stabilizing or increasing muscle strength, muscle control, or motor skills; reducing muscle fatigue; stabilizing or improving diaphragm function; stabilizing or improving cardiac output, etc. In non-limiting examples, the term "treat" in the context of cancer can encompass slowing or inhibiting the rate of cancer growth, or reducing the likelihood of recurrence, compared to not having the treatment. In some embodiments, the treatment encompasses resulting in some detectable degree of cancer cell death in the patient. The treatment or amelioration of symptoms can be based on objective or subjective parameters, including the results of an examination by a physician. Accordingly, the term "treating" includes the administration of the compositions of the present disclosure to alleviate, or to arrest or inhibit development of the symptoms or conditions associated with disease or condition (e.g., DMD, BMD, or cancer). The term "therapeutic effect" refers to the amelioration, reduction, or elimination of the disease or condition, symptoms of the disease or condition, or side effects of the disease or condition in the subject. The term "therapeutically effective" refers to an amount of the composition that results in a therapeutic effect and can be readily determined.

As indicated above, the method can comprise administering to the subject a therapeutically effective amount of the disclosed composition. As indicated, the nucleic acid payload is typically configured for expression in the target cell, *e.g.,* being operatively linked to an appropriate promoter. An effective amount is an amount that provides results in a beneficial change in phenotype. A benefit to having a cell specific promoter, e.g., a muscle specific promoter, is that it will confer additional specificity to that already provided by the GLUT4 targeting of the binding domain.

In some embodiments, the method of treatment is combined or coordinated with other therapeutic strategies. Any other therapeutic strategy addressing conditions associated with mutated or aberrant dystrophin expression is contemplated in this combinatorial aspect. In the context of cancers expressing GLUT4, the other cancer strategy can be a cancer immunotherapy that utilizes immunomodulatory compositions (*e*.*g*., antibodies, immune cells, cytokines, etc.), which may boost the subject's own immune response against the cancer target. Such immune-therapies include adoptive immune cell therapies, including CAR T-cells, immune checkpoint inhibitor therapies, cancer vaccines, and the like.

The methods of treatment can incorporate the disclosed composition to address a number of conditions associated with cells expressing GLUT4. For example, the diseases that can be addressed with administration of the disclosed composition include skeletal muscle diseases such as: the muscular dystrophies (myotonic dystrophy (Steinert disease), Duchenne muscular dystrophy, Becker muscular dystrophy, limb-girdle muscular dystrophies, facioscapulohumeral muscular dystrophy, congenital muscular dystrophy, oculophyaryngeal muscular dystrophy, distal muscular dystrophy and Emery-Dreifuss muscular dystrophy), other myopathies such as the inflammatory myopathies (for example polymyositis, dermatomyositis and inclusion-body myositis), myopathies due to endocrine abnormalities (hyperthyroid and hypothyroid myopathies), diseases causing muscle atrophy, such as cancer cachexia and sarcopenia, myotonia congenital, paramyotonia congenital, central core disease, nemaline myopathy, myotubular myopathy periodic paralysis, RYR-1 myopathies, and the inflammatory myopathies (for example polymyositis, dermatomyositis and inclusion-body myositis). The disclosed method can also be applied to treat the motor neuron diseases (for example amyotrophic lateral sclerosis (ALS), infantile progressive spinal muscular atrophy (Type 1 - Werdnig-Hoffmann disease), intermediate spinal muscular atrophy (Type 2), juvenile spinal muscular atrophy (Type 3 - Kugelberg-Welander disease), adult spinal muscular atrophy (Type 4) and spinal-bulbar muscular atrophy (Kennedy disease)), diseases of the neuromuscular junction such as myasthenia gravis, Lambert-Eaton (myasthenic) syndrome and congenital myasthenic syndromes, diseases of the peripheral nerves such as Charcot-Marie-Tooth disease, Friedreich's ataxia and Derjerine-Sottas disease, cardiomyopathies, metabolic diseases affecting muscle for example phosphorylase deficiency (McArdle disease), acid maltase deficiency (Pompe disease), phosphofructokinase deficiency (Tarui disease), debrancher enzyme deficiency (Cori or Forbes disease), mitochondrial myopathy, carnitine deficiency, carnitine palmityl transferase deficiency, phosphoglycerate kinase deficiency, phosphoglycerate mutase deficiency, lactate dehydrogenase deficiency and myoadenylate deaminase deficiency), and diseases affecting adipose tissue such as diabetes, adipose dolorosa, congenital generalized lipodystrophy (Berardinelli-Seip congenital lipodystrophy), familial partial lipodystrophy, familial lipoprotein lipase deficiency, neutral lipid storage disease and Chanarin-Dorfman syndrome. Because GLUT4 is expressed in the hippocampus and cerebellum, brain disorders including Alzheimer's disease, ataxia telangiectasia, NBIA (neurodegeneration with brain iron accumulation), Fahr's syndrome, Barth syndrome, and Parkinson's disease can be treated using the disclosed compositions. Finally, since GLUT4 is overexpressed in several cancers, the disclosed compositions can be used to target and treat malignant melanoma and prostate cancer, as well as cancers affecting muscle such as rhabdomyosarcoma.

In a specific aspect, the disclosure provides a method of treating a disease or condition in a subject with a genetic mutation in a gene encoding dystrophin protein. The method comprises administering to a subject a therapeutically effective amount of a composition comprising:
a binding domain that specifically binds glucose transporter 4 ("GLUT4") protein, and
a therapeutic payload conjugated to the binding domain, wherein the therapeutic payload comprises a nucleic acid with a promoter sequence operatively linked to a sequence encoding a dystrophin protein, or a functional fragment thereof.

In some embodiments, the subject has or is a genetic carrier of Duchenne muscular dystrophy ("DMD"). As described above, Duchenne muscular dystrophy (DMD) is a debilitating sex-linked disease resulting from aberrant dystrophin protein (or aberrant dystrophin protein expression). As a sex-linked disease, DMD mostly affects males and typically manifests first with muscle loss in the legs and pelvis, followed by the arms. Cases can progress to where the subject experiences difficulty breathing and poor cardiac output. Affected subjects ultimately have a significantly shortened life-span due to the extensive loss and degradation. In some embodiments, the subject has or is a genetic carrier of Becker muscular dystrophy ("BMD"). Becker muscular dystrophy ("BMD") is related to DMD in that it also results from mutations in the dystrophin gene. Becker muscular dystrophy ("BMD"), however, follows a milder course of symptoms compared to DMD likely because there are relatively higher levels of remaining dystrophin function in BDM subjects compared to DMD subjects due to the location of the mutation in the dystrophin gene.

Exemplary elements of the composition are described in more detail above and are applicable to this aspect. In some embodiments, the binding domain is an antibody, or fragment or derivative thereof, that specifically binds to GLUT4. Such antibodies, or fragments or derivatives thereof, are described in more detail above. In some embodiments, the antibody, or fragment or derivative thereof, specifically binds to the first extracellular domain of GLUT4. The domain and particular epitopes therein are described in more detail above. In some embodiments, the binding domain specifically binds to a first extracellular loop domain of GLUT4. In some embodiments, the first extracellular loop domain of GLUT4 comprises an amino acid sequence with at least about 80% sequence identity (e.g., at least about 80%, at least about 85%, at least about 90%, at least about 92%, at least about 94%, at least about 96%, at least about 98%, and 100% sequence identity) to the sequence set forth in SEQ ID NO:2, which is a subdomain of a human GLUT4 first extracellular loop domain. In some embodiments, the GLUT4 first extracellular loop domain comprises an amino acid sequence with at least about 80% sequence identity (e.g., at least about 80%, at least about 85%, at least about 90%, at least about 92%, at least about 94%, at least about 96%, at least about 98%, and 100% sequence identity) to the sequence set forth in SEQ ID NO: 1, which is a representative human GLUT4 first extracellular loop domain. In some embodiments, the binding domain specifically binds to a subdomain of the first extracellular loop domain of GLUT4 that comprises a sequence with at least 80% sequence identity *(e.g.,* at least about 80%, at least about 85%, at least about 90%, at least about 92%, at least about 94%, at least about 96%, at least about 98% sequence identity) to the sequence set forth in SEQ ID NO:2.

In some embodiments, the binding domain is a monoclonal antibody that binds to GLUT4 as described above. In some embodiments, the binding domain is an antibody fragment or antibody derivative. The antibody fragment or antibody derivative can be or comprise a single chain antibody, an Fab fragment, an F(ab)2 fragment, a V_{H}H fragment, a V_{NAR} fragment, or a nanobody. The single chain antibody can be a single chain variable fragment (scFv) or a single-chain Fab fragment (scFab).

In some embodiments, the antibody, or fragment or derivative thereof, is biotinylated.

The nucleic acid in this method aspect can be any nucleic acid payload as described above. For example, the nucleic acid is in linear form, plasmid form, or minicircle form.

In some embodiments, the composition further comprises a histone protein or a plurality of histone proteins associated with the nucleic acid. In some embodiments, the histone protein or at least a portion of the plurality of histone proteins is biotinylated.

In some embodiments, the composition further comprises a liposome associated with the nucleic acid. Elements of liposomes are described in more detail above and are applicable to this aspect of the disclosure. In some embodiments, the liposome comprises one or more lipids selected from DC-Cholesterol·HCl,3b-[N-(N',N'-dimethylaminoethane)-caramoyl]cholesterol hydrochloride ("DC-Chol"), 1,2-dioleoyl-sn-glycero-3-phosphoethanolamine ("DOPE"), 1,2-di-O-octadecenyl-3-trimethylammonium ("DOTMA"), N-[1-(2,3-Dioleoyloxy)propyl]-N,N,N-trimethylammonium propane ("DOTAP"), dimethyldioctadecylammonium bromide ("DDAB"), 1,2-dimyristoyl-3-trimethylammonium-propane ("DMTAP"). 1,2-dioleoyl-3-dimethylammonium-propane ("DODAP"), cholesteryl hemisuccinate ("CHEMS") and cholesterol ("CHOL"), 2,3-dioleyloxy-N-[2(sperminecarboxamido)ethyl]-N,N-dimethyl-1-propanaminium ("DOSPA"), Di-octadecyl-amido-glycyl-spermine ("DOGS"), dioleoylphosphatidylcholine ("DOPC"), and the like. In some embodiments, the liposome further comprises polyethylene glycol ("PEG").

In some embodiments, the composition comprises a plurality of cationic peptide moieties associated with the nucleic acid, thereby resulting in condensed nucleic acid. In some embodiments, the cationic moieties are or comprise poly-L-Lysine ("PLL"), linear Polyethylenimine ("LPEI"), branched Polyethylenimine ("BPEI"), chitosan, spermidine and spermine, Polyamidoamine ("PAMAM"), poly(2-dimethylaminoethyl methacrylate) ("PDMAEMA"), Poly(beta-amino ester)s ("PBAEs"), poly {N-[N-(2-aminoethyl)-2-aminoethyl]aspartamide ("PAsp(DET)"), poly(2-aminoethyl ethylene phosphate ("PPEEA"), and the like. In some embodiments, at least a portion of the plurality the cationic peptide moieties is biotinylated.

In some embodiments, the composition further comprises albumin. In some embodiments, at least a portion of the albumin is biotinylated.

In some embodiments, the antibody, or fragment or derivative thereof, is biotinylated and the composition further comprises neutravidin.

In some embodiments, the encoded dystrophin protein is a substantially full length human dystrophin protein. In some embodiments, the full length human dystrophin protein has an amino acid sequence with at least 80% sequence identity to the sequence set forth in SEQ ID NO:5.

The subject can be a mammal, such as a human, non-human primate, rat, mouse, rabbit, cat, dog, and the like. In certain embodiments, the subject is a human.

### Formulation and administration

The disclosure also encompasses formulations appropriate for methods of administration for application to *in vivo* therapeutic settings in subjects *(e.g.,* mammalian subjects with DMD, BMD, cancer, or the conditions described above). According to skill and knowledge common in the art, the disclosed compositions can be formulated with appropriate carriers and non-active binders, and the like, for administration to target specific cells. Because the compositions comprise binding domains that confer target cell specificity, the compositions can be formulated for direct or systemic administration according to skill and knowledge in the art.

### Illustrative Anti-GLUT4 antibodies.

Anti-GLUT4 antibodies that bind the extracellular domain of human GLUT4 are known (see, e.g., Tucker et al, Proc. Natl. Acad. Sci. 115:E4990-E4999, published online May 16, 2018). In a further aspect, provided herein are monoclonal antibodies that bind a human GLUT4 peptide GRQGPEGPSSI (SEQ ID NO:2). Thus, in certain embodiments, the disclosure provides an anti-GLUT4 antibody, or fragment or derivative thereof comprising a heavy chain variable region (V_{H}) and a light chain variable region (V_{L}). In some aspects, the V_{H} comprises a CDR1 comprising a sequence (D/E)Y(S/T)(I/M)H, a CDR2 comprising a sequence WINTE(S/T)G(D/E)X₁(T/S)YADDFKG, and a CDR3 comprising a sequence RX₂X₃Y; and the V_{L} comprises a CDR1 comprising a sequence (R/K)(A/S)SQS(L/V)X₄X₅ (N/S), a CDR2 comprising a sequence (V/A)SNR(F/Y)(S/T), and a CDR3 comprising a sequence QDX₆ X₇X₈P T. In some embodiments, X₁ is P or T; X₂, is A, F, S, or G; X₃ is A, D, E, or G; X₄ is S, V, N, or T; X₅ is N, H, R, K, or T; X₆ is R, Y, S, T, or K; X₇ is H N, E, T, Y, or S; or X₈ is V, S, L or I.

Accordingly, in some embodiments, the anti-GLUT4 antibody, or fragment or derivative thereof comprises at least one, two, three, four, five, or six CDRs selected from (a) a VH-CDR1 comprising a sequence (D/E)Y(S/T)(I/M)H; (b) a VH-CDR2 comprising a sequence WINTE(S/T)G(D/E)X₁(T/S)YADDFKG; (c) a VH-CDR3 comprising a sequence RX₂X₃Y; (d) a VL-CDR1 comprising a sequence (R/K)(A/S)SQS(L/V)X₄X₅ (N/S); (e) a VL-CDR2 comprising a sequence (V/A)SNR(F/Y)(S/T); and (f) a VL-CDR3 comprising a sequence QDX₆ X₇X₈P T. In some embodiments, X₁ is P or T; X₂, is A, F, S, or G; X₃ is A, D, E, or G; X₄ is S, V, N, or T; X₅ is N, H, R, K, or T; X₆ is R, Y, S, T, or K; X₇ is H N, E, T, Y, or S; or X₈ is V, S, L or I.

In some embodiments, the anti-GLUT4 antibody, or fragment or derivative thereof comprises at least one, two, three, four, five, or six CDRs selected from (a) a VH-CDR1 having a sequence selected from SEQ ID NOs:10-17; (b) a VH-CDR2 having a sequence selected from SEQ ID NOs:18-25; (c) a VH-CDR3 having a sequence selected from LDF and SEQ ID NOs:27-33; (d) a VL-CDR1 having a sequence selected from SEQ ID NOs:34-41; (e) a VL-CDR2 having a sequence selected from SEQ ID NOs:42-49; and (f) a VL-CDR3 having a sequence selected from SEQ ID NOs:50-57.

In some embodiments, the disclosure provides an anti-GLUT4 antibody, or fragment or derivative thereof comprising a heavy chain variable region (V_{H}) and a light chain variable region (V_{L}). In some embodiments, the V_{H} comprises a VH-CDR1 having a sequence selected from SEQ ID NOs:10-17, a VH-CDR2 having a sequence selected from SEQ ID NOs:18-25, and a VH-CDR3 having a sequence selected from LDF and SEQ ID NOs:27-33; and the V_{L} comprises a VL-CDR1 having a sequence selected from SEQ ID NOs:34-41, a VL-CDR2 having a sequence selected from SEQ ID NOs:42-49, and a VL-CDR3 having a sequence selected from SEQ ID NOs:50-57.

In some embodiments, the V_{H} comprises a VH-CDR1 having a sequence set forth in SEQ ID NO 10, a VH-CDR2 having a sequence set forth in SEQ ID NO:18, and a VH-CDR3 having a sequence LDF; and the V_{L} comprises a VL-CDR1 having a sequence set forth in SEQ ID NO:34, a VL-CDR2 having a sequence set forth in SEQ ID NO:42, and a VL-CDR3 having a sequence set forth in SEQ ID NO:50.

In some embodiments, the V_{H} comprises a VH-CDR1 having a sequence set forth in SEQ ID NO:11, a VH-CDR2 having a sequence set forth in SEQ ID NO:19, and a VH-CDR3 having a sequence set forth in SEQ ID NO:27; and the V_{L} comprises a VL-CDR1 having a sequence set forth in SEQ ID NO:35, a VL-CDR2 having a sequence set forth in SEQ ID NO:43, and a VL-CDR3 having a sequence set forth in SEQ ID NO:51.

In some embodiments, the V_{H} comprises a VH-CDR1 having a sequence set forth in SEQ ID NO:12, a VH-CDR2 having a sequence set forth in SEQ ID NO:20, and a VH-CDR3 having a sequence set forth in SEQ ID NO:28; and the V_{L} comprises a VL-CDR1 having a sequence set forth in SEQ ID NO:36, a VL-CDR2 having a sequence set forth in SEQ ID NO:44, and a VL-CDR3 having a sequence set forth in SEQ ID NO:52.

In some embodiments, the V_{H} comprises a VH-CDR1 having a sequence set forth in SEQ ID NO 13, a VH-CDR2 having a sequence set forth in SEQ ID NO:21, and a VH-CDR3 having a sequence set forth in SEQ ID NO:29; and the V_{L} comprises a VL-CDR1 having a sequence set forth in SEQ ID NO:37, a VL-CDR2 having a sequence set forth in SEQ ID NO:45, and a VL-CDR3 having a sequence set forth in SEQ ID NO:53.

In some embodiments, the V_{H} comprises a VH-CDR1 having a sequence set forth in SEQ ID NO:14, a VH-CDR2 having a sequence set forth in SEQ ID NO:22, and a VH-CDR3 having a sequence set forth in SEQ ID NO:30; and the V_{L} comprises a VL-CDR1 having a sequence set forth in SEQ ID NO:38, a VL-CDR2 having a sequence set forth in SEQ ID NO:46, and a VL-CDR3 having a sequence set forth in SEQ ID NO:54

In some embodiments, the V_{H} comprises a VH-CDR1 having a sequence set forth in SEQ ID NO:15, a VH-CDR2 having a sequence set forth in SEQ ID NO:23, and a VH-CDR3 having a sequence set forth in SEQ ID NO:31; and the V_{L} comprises a VL-CDR1 having a sequence set forth in SEQ ID NO:39, a VL-CDR2 having a sequence set forth in SEQ ID NO:47, and a VL-CDR3 having a sequence set forth in SEQ ID NO:55

In another embodiment, the V_{H} comprises a VH-CDR1 having a sequence set forth in SEQ ID NO:16, a VH-CDR2 having a sequence set forth in SEQ ID NO:24, and a VH-CDR3 having a sequence set forth in SEQ ID NO:32; and the V_{L} comprises a VL-CDR1 having a sequence set forth in SEQ ID NO:40, a VL-CDR2 having a sequence set forth in SEQ ID NO:48, and a VL-CDR3 having a sequence set forth in SEQ ID NO:56.

In yet another embodiment, the V_{H} comprises a VH-CDR1 having a sequence set forth in SEQ ID NO:17, a VH-CDR2 having a sequence set forth in SEQ ID NO:25, and a VH-CDR3 having a sequence set forth in SEQ ID NO:33; and the V_{L} comprises a VL-CDR1 having a sequence set forth in SEQ ID NO:41, a VL-CDR2 having a sequence set forth in SEQ ID NO:49, and a VL-CDR3 having a sequence set forth in SEQ ID NO:57.

In certain embodiments, substitutions, insertions, or deletions may occur within one or more CDRs without substantially reducing the ability of the antibody to bind antigen. For example, conservative alterations (e.g., conservative substitutions as provided herein) that do not substantially reduce binding affinity may be made in CDRs. In certain embodiments, each CDR provided above either is unaltered, or contains one, two, three or four amino acid substitutions.

In some embodiments, the anti-GLUT4 antibody, or fragment or derivative thereof further comprises CDRs as defined in the preceding paragraphs in this section and a framework regions sequence having at least 90% identity, or at least 95% identity to a human immunoglobulin framework sequences. In some embodiments, each of framework region 1 (FR1), framework region 2 (FR2), framework region 3 (FR3), and framework region 4 (FR4) have at least 90% identity, or at least 95% identity to a corresponding human FR1, FR2, FR3, or FR4 sequence.

An antibody as defined above that binds a human GLUT4 peptide of amino acid sequence GRQGPEGPSSI (SEQ ID NO:2) can be used to deliver a payload as described in any of the embodiments detailed herein.

### Illustrative embodiments

Illustrative embodiments include, but are not limited, to the following:
In some embodiments, the disclosure provides a composition comprising an antibody, or fragment or derivative thereof, that specifically binds to glucose transporter 4 ("GLUT4") protein, and a therapeutic payload conjugated to the antibody, or fragment or derivative thereof. In one embodiment, the antibody, or fragment or derivative thereof, specifically binds to a first extracellular loop domain of GLUT4. In some aspects, the first extracellular loop domain of GLUT4 comprises an amino acid sequence with at least 80% identity to the sequence INAPQKVIEQSYNETWLGRQGPEGPSSIPPGTLTTL(SEQ ID NO: 1). In one aspect, the first extracellular loop domain of GLUT4 comprises an amino acid sequence with at least 80% identity to the sequence GRQGPEGPSSI (SEQ ID NO:2) In some aspects, the antibody, or fragment or derivative thereof, specifically binds to a subdomain of the first extracellular loop domain of GLUT4 that comprises an amino acid sequence with at least 80% identity to the sequence GRQGPGGPDSI(SEQ ID NO:4). In one aspect, the antibody, or fragment or derivative thereof, specifically binds to a subdomain of the first extracellular loop domain of GLUT4 that comprises a sequence with at least 80% identity to the sequence GRQGPEGPSSI (SEQ ID NO:2).

In some embodiments, the antibody is a monoclonal antibody. In one embodiment, the antibody fragment or antibody derivative is or comprises a single chain antibody, an Fab fragment, an F(ab)2 fragment, a V_{H}H fragment, a V_{NAR} fragment, or a nanobody. In some aspects, the single-chain antibody is a single chain variable fragment (scFv), or a single-chain Fab fragment (scFab).

In some embodiments, the antibody, or fragment or derivative thereof, is biotinylated.

In some embodiments, the therapeutic payload comprises a nucleic acid, a protein or peptide, a lipid, a small molecule pharmaceutical, or a radioisotope. In one embodiment, the therapeutic payload comprises a nucleic acid, wherein the nucleic acid is selected from DNA, mRNA, siRNA, and shRNA. In one embodiment, the therapeutic payload comprises a nucleic acid, wherein the nucleic acid comprises an open reading frame operatively linked to a promoter sequence. In one embodiment, the therapeutic payload comprises a nucleic acid in linear form, a nucleic acid in plasmid form, or a nucleic acid in minicircle form.

In some embodiments, the composition further comprises a histone protein or a plurality of histone proteins associated with the nucleic acid. In one aspect, the histone protein or at least a portion of the plurality of histone proteins is biotinylated.

In some embodiments, the composition further comprises a liposome associated with the nucleic acid. In one aspect, the liposome comprises one or more lipids selected from DC-Cholesterol·HCl,3b-[N-(N',N'-dimethylaminoethane)-caramoyl]cholesterol hydrochloride ("DC-Chol"), 1,2-dioleoyl-sn-glycero-3-phosphoethanolamine ("DOPE"), 1,2-di-O-octadecenyl-3-trimethylammonium ("DOTMA"), N-[1-(2,3-Dioleoyloxy)propyl]-N,N,N-trimethylammonium propane ("DOTAP"), dimethyldioctadecylammonium bromide ("DDAB"), 1,2-dimyristoyl-3-trimethylammonium-propane ("DMTAP"), 1,2-dioleoyl-3-dimethylammonium-propane ("DODAP"), cholesteryl hemisuccinate ("CHEMS") and cholesterol ("CHOL"), 2,3-dioleyloxy-N-[2(sperminecarboxamido)ethyl]-N,N-dimethyl-1-propanaminium ("DOSPA"), Di-octadecyl-amido-glycyl-spermine ("DOGS"), dioleoylphosphatidylcholine ("DOPC"), and the like. In one aspect, the liposome further comprises polyethylene glycol ("PEG").

In some embodiments, the composition further comprises a plurality of cationic peptide moieties associated with the nucleic acid, thereby resulting in condensed nucleic acid. In one embodiment, the cationic peptide moieties are or comprise poly-L-Lysine ("PLL"), linear *Polyethylenimine* ("LPEI"), branched *Polyethylenimine* ("BPEI"), chitosan, spermidine and spermine, Polyamidoamine ("PAMAM"), poly(2-dimethylaminoethyl methacrylate) (*"PDMAEMA*")*,* Poly(beta-amino ester)s (*"PBAEs"*), poly{*N*-[*N*-(2-aminoethyl)-2-aminoethyl]aspartamide ("PAsp(DET)"), poly(2-aminoethyl ethylene phosphate ("PPEEA"), and the like. In one aspect, at least a portion of the plurality the cationic peptide moieties is biotinylated.

In some embodiments, the composition of the present disclosure further comprises albumin. In one embodiment, at least a portion of the albumin is biotinylated.

In some embodiment, the antibody, or fragment or derivative thereof, is biotinylated and the composition further comprises neutravidin.

In some embodiment, the therapeutic payload comprises a nucleic acid with a promoter sequence operatively linked to a sequence encoding a dystrophin protein, or a functional fragment thereof. In one embodiment, the dystrophin protein is a substantially full-length human dystrophin protein. In one aspect, the full-length human dystrophin protein has an amino acid sequence with at least 80% sequence identity to the sequence set forth in SEQ ID NO:5.

The disclosure further provides a method of targeting a therapeutic payload for delivery to a cell that expresses GLUT4, the method comprising contacting the cell with the composition. In some embodiments, the method further comprises contacting the cell with insulin prior to contacting the cell with the composition. In one embodiment, the cell is a skeletal muscle cell, a cardiac muscle cell, a smooth muscle cell, an adipose tissue cell, a hippocampal cell, or a cerebellum cell. In one aspect, the cell is a melanoma cell, prostate cancer cell, cancer cell of muscle tissue, such as rhabdomyosarcoma cell, or a breast cancer cell.

In another aspect, the disclosure includes method of treating a disease or condition in a subject with a genetic mutation in a gene encoding dystrophin protein, comprising administering to a subj ect a therapeutically effective amount of a composition comprising: an antibody, or fragment or derivative thereof, that specifically binds glucose transporter 4 ("GLUT4") protein, and a therapeutic payload conjugated to the antibody, or fragment or derivative thereof, wherein the therapeutic payload comprises a nucleic acid with a promoter sequence operatively linked to a sequence encoding a dystrophin protein, or a functional fragment thereof. In some embodiments, the subject has or is a genetic carrier of Duchenne muscular dystrophy ("DMD") or Becker muscular dystrophy ("BMD").

In some embodiments, the antibody, or fragment or derivative thereof, employed in the method of treating a disease or condition described in the preceding two paragraphs specifically binds to a first extracellular loop domain of GLUT4. In one embodiment, the first extracellular loop domain of GLUT4 comprises an amino acid sequence with at least 80% identity to the sequence INAPQKVIEQSYNETWLGRQGPEGPSSIPPGTLTTL (SEQ ID NO: 1). In some aspects, the antibody, or fragment or derivative thereof, specifically binds to a subdomain of the first extracellular loop domain of GLUT4 that comprises a sequence with at least 80% identity to the sequence GRQGPEGPSSI (SEQ ID NO:2). In some embodiments, the antibody is a monoclonal antibody. In one embodiment, the antibody fragment or antibody derivative is or comprises a single chain antibody, an Fab fragment, an F(ab)2 fragment, a V_{H}H fragment, a V_{NAR} fragment, or a nanobody. In some aspects, the single-chain antibody is a single chain variable fragment (scFv), or a single-chain Fab fragment (scFab). In some embodiments, the antibody, or fragment or derivative thereof, is biotinylated. In some embodiments, the nucleic acid is in linear form, plasmid form, or minicircle form. In one embodiment, the composition further comprises a histone protein or a plurality of histone proteins associated with the nucleic acid. In one aspect, the histone protein or at least a portion of the plurality of histone proteins is biotinylated. In some embodiments, the composition further comprises a liposome associated with the nucleic acid. In one embodiment, the liposome comprises one or more lipids selected from DC-Cholosterol·HCl,3b-[N-(N',N'-dimethylaminoethane)-caramoyl]cholesterol hydrochloride ("DC-Chol"), 1,2-dioleoyl-sn-glycero-3-phosphoethanolamine ("DOPE"), 1,2-di-O-octadecenyl-3-trimethylammonium ("DOTMA"), N-[1-(2,3-Dioleoyloxy)propyl]-N,N,N-trimethylammonium propane ("DOTAP"), dimethyldioctadecylammonium bromide ("DDAB"), 1,2-dimyristoyl-3-trimethylammonium-propane ("DMTAP"), 1,2-dioleoyl-3-dimethylammonium-propane ("DODAP"), cholesteryl hemisuccinate ("CHEMS") and cholesterol ("CHOL"), 2,3-dioleyloxy-N-[2(sperminecarboxamido)ethyl]-N,N-dimethyl-1-propanaminium ("DOSPA"), Di-octadecyl-amido-glycyl-spermine ("DOGS"), dioleoylphosphatidylcholine ("DOPC"), and the like. In one aspect, the liposome further comprises polyethylene glycol ("PEG"). In some embodiments, the composition further comprises a plurality of cationic peptide moieties associated with the nucleic acid, thereby resulting in condensed nucleic acid. In one embodiment, the cationic peptide moieties are or comprise poly-L-Lysine ("PLL"), linear *Polyethylenimine ("LPEI"), branched Polyethylenimine* ("BPEI"), chitosan, spermidine and spermine, Polyamidoamine ("PAMAM"), poly(2-dimethylaminoethyl methacrylate) *("PDMAEMA* "), Poly(beta-amino ester)s *("PBAEs* "), poly{*N*-[*N*-(2-aminoethyl)-2-aminoethyl]aspartamide ("PAsp(DET)"), poly(2-aminoethyl ethylene phosphate ("PPEEA"), and the like. In one aspect, at least a portion of the plurality the cationic peptide moieties is biotinylated. In some embodiments, the composition further comprises albumin. In one embodiment, at least a portion of the albumin is biotinylated. In some embodiments, the antibody, or fragment or derivative thereof, is biotinylated and the composition further comprises neutravidin. In some embodiments, the encoded dystrophin protein is a substantially full-length human dystrophin protein. In one embodiment, the full-length human dystrophin protein has an amino acid sequence with at least 80% sequence identity to the sequence (SEQ ID NO:5). In some embodiments, the subject is a human.

In still another aspect, the disclosure provides an anti-GLUT4 antibody, or fragment or derivative thereof comprising a heavy chain variable region (V_{H}) and a light chain variable region (V_{L}), wherein: (a) the V_{H} comprises a CDR1 comprising a sequence (D/E)Y(S/T)(I/M)H, a CDR2 comprising a sequence WINTE(S/T)G(D/E)X₁(T/S)YADDFKG, and a CDR3 comprising a sequence RX₂X₃Y; and (b) the V_{L} comprises a CDR1 comprising a sequence (R/K)(A/S)SQS(L/V)X₄X₅ (N/S), a CDR2 comprising a sequence (V/A)SNR(F/Y)(S/T), and a CDR3 comprising a sequence QDX₆ X₇X₈P T In some embodiments, X₁ is P or T; X₂, is A, F, S, or G; X₃ is A, D, E, or G; X₄ is S, V, N, or T; X₅ is N, H, R, K, or T; X₆ is R, Y, S, T, or K; X₇ is H N, E, T, Y, or S; or X₈ is V, S, L or I.

In yet another aspect, the disclosure includes an anti-GLUT4 antibody, or fragment or derivative thereof comprising a heavy chain variable region (V_{H}) and a light chain variable region (V_{L}), wherein: (a) the V_{H} comprises a VH-CDR1 having a sequence selected from SEQ ID NOs:10-17, a VH-CDR2 having a sequence selected from SEQ ID NOs:18-25, and a VH-CDR3 having a sequence selected from LDF and SEQ ID NOs:27-33; and (b) the V_{L} comprises a VL-CDR1 having a sequence selected from SEQ ID NOs:34-41, a VL-CDR2 having a sequence selected from SEQ ID NOs:42-49, and a VL-CDR3 having a sequence selected from SEQ ID NOs:50-57.

In some embodiments, the anti-GLUT4 antibody, or fragment or derivative thereof comprising a heavy chain variable region (V_{H}) and a light chain variable region (V_{L}), wherein: (a) the V_{H} comprises a VH-CDR1 having a sequence set forth in SEQ ID NO 10, a VH-CDR2 having a sequence set forth in SEQ ID NO:18, and a VH-CDR3 having a sequence LDF; and the V_{L} comprises a VL-CDR1 having a sequence set forth in SEQ ID NO:34, a VL-CDR2 having a sequence set forth in SEQ ID NO:42, and a VL-CDR3 having a sequence set forth in SEQ ID NO:50; (b) the V_{H} comprises a VH-CDR1 having a sequence set forth in SEQ ID NO:11, a VH-CDR2 having a sequence set forth in SEQ ID NO:19, and a VH-CDR3 having a sequence set forth in SEQ ID NO:27; and the V_{L} comprises a VL-CDR1 having a sequence set forth in SEQ ID NO:35, a VL-CDR2 having a sequence set forth in SEQ ID NO:43, and a VL-CDR3 having a sequence set forth in SEQ ID NO:51; (c) the V_{H} comprises a VH-CDR1 having a sequence set forth in SEQ ID NO:12, a VH-CDR2 having a sequence set forth in SEQ ID NO:20, and a VH-CDR3 having a sequence set forth in SEQ ID NO:28; and the V_{L} comprises a VL-CDR1 having a sequence set forth in SEQ ID NO:36, a VL-CDR2 having a sequence set forth in SEQ ID NO:44, and a VL-CDR3 having a sequence set forth in SEQ ID NO:52; (d) the V_{H} comprises a VH-CDR1 having a sequence set forth in SEQ ID NO 13, a VH-CDR2 having a sequence set forth in SEQ ID NO:21, and a VH-CDR3 having a sequence set forth in SEQ ID NO:29; and the V_{L} comprises a VL-CDR1 having a sequence set forth in SEQ ID NO:37, a VL-CDR2 having a sequence set forth in SEQ ID NO:45, and a VL-CDR3 having a sequence set forth in SEQ ID NO:53; (e) the V_{H} comprises a VH-CDR1 having a sequence set forth in SEQ ID NO:14, a VH-CDR2 having a sequence set forth in SEQ ID NO:22, and a VH-CDR3 having a sequence set forth in SEQ ID NO:30; and the V_{L} comprises a VL-CDR1 having a sequence set forth in SEQ ID NO:38, a VL-CDR2 having a sequence set forth in SEQ ID NO:46, and a VL-CDR3 having a sequence set forth in SEQ ID NO:54; (f) the V_{H} comprises a VH-CDR1 having a sequence set forth in SEQ ID NO:15, a VH-CDR2 having a sequence set forth in SEQ ID NO:23, and a VH-CDR3 having a sequence set forth in SEQ ID NO:31; and the V_{L} comprises a VL-CDR1 having a sequence set forth in SEQ ID NO:39, a VL-CDR2 having a sequence set forth in SEQ ID NO:47, and a VL-CDR3 having a sequence set forth in SEQ ID NO:55; (g) the V_{H} comprises a VH-CDR1 having a sequence set forth in SEQ ID NO: 16, a VH-CDR2 having a sequence set forth in SEQ ID NO:24, and a VH-CDR3 having a sequence set forth in SEQ ID NO:32; and the V_{L} comprises a VL-CDR1 having a sequence set forth in SEQ ID NO:40, a VL-CDR2 having a sequence set forth in SEQ ID NO:48, and a VL-CDR3 having a sequence set forth in SEQ ID NO:56; or (h) the V_{H} comprises a VH-CDR1 having a sequence set forth in SEQ ID NO:17, a VH-CDR2 having a sequence set forth in SEQ ID NO:25, and a VH-CDR3 having a sequence set forth in SEQ ID NO:33; and the V_{L} comprises a VL-CDR1 having a sequence set forth in SEQ ID NO:41, a VL-CDR2 having a sequence set forth in SEQ ID NO:49, and a VL-CDR3 having a sequence set forth in SEQ ID NO:57.

In some embodiments, the disclosure provides a composition comprising an antibody, or fragment or derivative thereof, of any one of the embodiments above described in the three preceding paragraphs, and a therapeutic payload conjugated to the antibody, or fragment or derivative thereof. In some embodiments, the antibody fragment or antibody derivative is or comprises a single chain antibody, an Fab fragment, an F(ab)2 fragment, a V_{H}H fragment, a V_{NAR} fragment, or a nanobody. In one aspect, the single-chain antibody is a single chain variable fragment (scFv), or a single-chain Fab fragment (scFab). In some embodiments, the therapeutic payload comprises a nucleic acid, a protein or peptide, a lipid, a small molecule pharmaceutical, or a radioisotope. In some embodiments, the therapeutic payload comprises a nucleic acid, wherein the nucleic acid is selected from DNA, mRNA, siRNA, and shRNA. In some embodiments, the therapeutic payload comprises a nucleic acid, wherein the nucleic acid comprises an open reading frame operatively linked to a promoter sequence. In some embodiments, the therapeutic payload comprises a nucleic acid in linear form, a nucleic acid in plasmid form, or a nucleic acid in minicircle form. In some embodiments, the composition further comprises a histone protein or a plurality of histone proteins associated with the nucleic acid. In some embodiments, the composition further comprises a liposome associated with the nucleic acid. In some embodiments, the composition further comprises a plurality of cationic peptide moieties associated with the nucleic acid, thereby resulting in condensed nucleic acid. In some embodiments, the therapeutic payload comprises a nucleic acid with a promoter sequence operatively linked to a sequence encoding a dystrophin protein, or a functional fragment thereof. In one embodiment, the dystrophin protein is a substantially full-length human dystrophin protein having an amino acid sequence with at least 80% sequence identity to the sequence set forth in SEQ ID NO:5.

In some embodiments, the disclosure provides a method of targeting a therapeutic payload for delivery to a cell that expresses GLUT4, the method comprising contacting the cell with the composition comprising an anti-GLUT4 antibody, or fragment or derivative thereof as described in the preceding paragraph. In some embodiments, the method further comprises contacting the cell with insulin prior to contacting the cell with the composition. In one embodiment, the cell is a skeletal muscle cell, a cardiac muscle cell, a smooth muscle cell, an adipose tissue cell, a hippocampal cell, or a cerebellum cell. In one embodiment, the cell is a melanoma cell, prostate cancer cell, cancer cell of muscle tissue, such as rhabdomyosarcoma cell, or a breast cancer cell. In some embodiments, the subject is a human.

In some embodiments, the disclosure provides a method of treating a disease or condition in a subject with a genetic mutation in a gene encoding dystrophin protein, comprising administering to a subject a therapeutically effective amount of a composition comprising an antibody as specified in any of the embodiments above. In some embodiment, the subject has or is a genetic carrier of Duchenne muscular dystrophy ("DMD") or Becker muscular dystrophy ("BMD"). In some embodiments, the encoded dystrophin protein has at least 80% sequence identity to SEQ ID NO:5. In some embodiments, the subject is a human.

### General definitions

Unless specifically defined herein, all terms used herein have the same meaning as they would to one skilled in the art of the present disclosure. Practitioners are particularly directed to Ausubel, F.M., et al. (eds.), Current Protocols in Molecular Biology, John Wiley & Sons, New York (2010), Coligan, J.E., et al. (eds.), Current Protocols in Immunology, John Wiley & Sons, New York (2010), Mirzaei, H. and Carrasco, M. (eds.), Modern Proteomics - Sample Preparation, Analysis and Practical Applications in Advances in Experimental Medicine and Biology, Springer International Publishing, 2016, and Comai, L, et al., (eds.), Proteomic: Methods and Protocols in Methods in Molecular Biology, Springer International Publishing, 2017, for definitions and terms of art.

For convenience, certain terms employed in this description and/or the claims are provided here. The definitions are provided to aid in describing particular embodiments and are not intended to limit the claimed invention, because the scope of the invention is limited only by the claims.

The use of the term "or" in the claims is used to mean "and/or" unless explicitly indicated to refer to alternatives only or the alternatives are mutually exclusive, although the disclosure supports a definition that refers to only alternatives and "and/or."

The words "a" and "an," when used in conjunction with the word "comprising" in the claims or specification, denotes one or more, unless specifically noted.

Unless the context clearly requires otherwise, throughout the description and the claims, the words "comprise," "comprising," and the like, are to be construed in an inclusive sense as opposed to an exclusive or exhaustive sense, which is to indicate, in the sense of "including, but not limited to." Words using the singular or plural number also include the plural and singular number, respectively. The word "about" indicates a number within range of minor variation above or below the stated reference number. For example, "about" can refer to a number within a range of 10%, 9%, 8%, 7%, 6%, 5%, 4%, 3%, 2%, or 1% above or below the indicated reference number.

As used herein, the term "polypeptide" or "protein" refers to a polymer in which the monomers are amino acid residues that are joined together through amide bonds. When the amino acids are alpha-amino acids, either the L-optical isomer or the D-optical isomer can be used, the L-isomers being preferred. The term polypeptide or protein as used herein encompasses any amino acid sequence and includes modified sequences such as glycoproteins. The term polypeptide is specifically intended to cover naturally occurring proteins, as well as those that are recombinantly or synthetically produced.

One of skill will recognize that individual substitutions, deletions or additions to a peptide, polypeptide, or protein sequence which alters, adds or deletes a single amino acid or a percentage of amino acids in the sequence is a "conservatively modified variant" where the alteration results in the substitution of an amino acid with a chemically similar amino acid. Conservative amino acid substitution tables providing functionally similar amino acids are well known to one of ordinary skill in the art. The following six groups are examples of amino acids that are considered to be conservative substitutions for one another:
(1) Alanine (A), Serine (S), Threonine (T),
(2) Aspartic acid (D), Glutamic acid (E),
(3) Asparagine (N), Glutamine (Q),
(4) Arginine (R), Lysine (K),
(5) Isoleucine (I), Leucine (L), Methionine (M), Valine (V), and
(6) Phenylalanine (F), Tyrosine (Y), Tryptophan (W).

Reference to sequence identity addresses the degree of similarity of two polymeric sequences, such as protein sequences. Determination of sequence identity can be readily accomplished by persons of ordinary skill in the art using accepted algorithms and/or techniques. Sequence identity is typically determined by comparing two optimally aligned sequences over a comparison window, where the portion of the peptide or polynucleotide sequence in the comparison window may comprise additions or deletions (e.g., gaps) as compared to the reference sequence (which does not comprise additions or deletions) for optimal alignment of the two sequences. The percentage is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. Various software driven algorithms are readily available, such as BLAST N or BLAST P to perform such comparisons.

Disclosed are materials, compositions, and components that can be used for, can be used in conjunction with, can be used in preparation for, or are products of the disclosed methods and compositions. It is understood that, when combinations, subsets, interactions, groups, etc., of these materials are disclosed, each of various individual and collective combinations is specifically contemplated, even though specific reference to each and every single combination and permutation of these compounds may not be explicitly disclosed. This concept applies to all aspects of this disclosure including, but not limited to, steps in the described methods. Thus, specific elements of any foregoing embodiments can be combined or substituted for elements in other embodiments. For example, if there are a variety of additional steps that can be performed, it is understood that each of these additional steps can be performed with any specific method steps or combination of method steps of the disclosed methods, and that each such combination or subset of combinations is specifically contemplated and should be considered disclosed. Additionally, it is understood that the embodiments described herein can be implemented using any suitable material such as those described elsewhere herein or as known in the art.

Publications cited herein and the subject matter for which they are cited are hereby specifically incorporated by reference in their entireties.

### EXAMPLES

The following is a description of a series of exemplary assays conducted on representative embodiments of the platform muscle-specific vector compositions to establish the utility of the platform design for efficiently delivering therapeutic payloads to muscle tissues and other tissues.

### Introduction

The disclosed non-viral vector platform utilizes a GLUT4 targeting antibody (or other binding agents (e.g., antibody fragment, peptides, receptors, etc.) capable of specific binding to an extracellular domain of GLUT4. The first extracellular loop is the largest, most accessible and likely immunogenic region for targeting GLUT4 extracellularly. This domain comprises amino acids 45-80 in the mouse GLUT4 sequence; INAPQKVIEQSYNATWLGRQGPGGPDSIPQGTLTTL (SEQ ID NO:3). The antibodies were generated against a peptide produced from amino acids 61-72 of this sequence; GRQGPGGPDSI (SEQ ID NO:4). The analogous region of the human sequence, GRQGPEGPSSI (SEQ ID NO:2), shares 83.3% homology to the mouse sequence. This sequence is predicted to not have any secondary structure, and was hypothesized to be amenable to generating antibodies that could bind to this domain of the native GLUT4 protein.

Using this platform, the GLUT4 antibody (or fragment thereof) can then be conjugated to a therapeutic gene or other drug using a wide variety of materials, such as proteins, peptides, lipids, liposomes, etc., to encapsulate the therapeutic agent, optimize its stability and bioavailability *in vivo,* and enhance endosomal escape and nuclear delivery (for nucleic acids) once inside the target cell. As demonstrated in the following experiments, the DNA size is not a limiting factor, allowing delivery of large genes such as full-length dystrophin (~14 Kb) and also use strong muscle-specific promoters to drive gene expression.

### In vitro cell culture experiments

For the initial *in vitro* cell culture experiments, a Biotin-NeutrAvidin system was used to conjugate targeting antibody to a cationic liposome containing the therapeutic plasmid. This embodiment of the vector platform for *in vitro* experiments is shown schematically in Fig. 3. First, the plasmid is mixed with Histone H1 (H-H1), some of which is biotinylated. H-H1 is a positively charged nuclear protein that binds to the negatively charged DNA and partly condenses it. Second, the GLUT4 antibody, which is also biotinylated, is added. Third, NeutrAvidin (deglycosylated avidin), a highly specific biotin binding protein with minimal non-specific binding compared to Avidin, is added to bind the biotinylated proteins. Fourth, the proteins and DNA are mixed with cationic liposomes comprising two lipids, DC-Chol (DC-Cholesterol·HCl,3b-[N-(N',N'-dimethylaminoethane)-caramoyl]cholesterol hydrochloride) and DOPE (1,2-dioleoyl-sn-glycero-3-phosphoethanolamine). The DC-Chol:DOPE molar ratio is 30:70. Each of the four incubation steps described above are at room temperature for 30 min.

In initial proof-of-concept experiments, this conjugate structure was assessed for whether it could be taken up by muscle cells *in vitro* and the protein encoded by the DNA plasmid expressed in the cells. These experiments used a commercially available rat myoblast cell line (L6-GLUT4myc), which overexpresses GLUT4 containing a myc tag (amino acid sequence AEEQKLISEEDLLK; SEQ ID NO:9) inserted into the first extracellular loop between amino acids 66 and 67. For the initial experiments using this cell line, a commercial antibody that binds specifically to the myc tag was biotinylated.

### Experiment 1

L6-GLUT4myc myoblasts in 6 well plates were transfected with the structure shown in Fig. 3. In this experiment, a plasmid was used containing a modified neuronal nitric oxide synthase construct (NOS-M), which included a C-terminal HA tag for detection and a RAS palmitoylation sequence for plasma membrane targeting (Rebolledo DL, et al. (2016). Sarcolemmal targeting of nNOSmu improves contractile function of mdx muscle. Hum Mol Genet 25, 158-166). This plasmid is driven by the human skeletal actin (HSA) promoter and is ~11 Kb in length. In this experiment, 10 µg of NOS-M plasmid were used for each well. The primary aims of this experiment were to test whether short-term incubation periods 15 to 45 minutes were sufficient for the antibody to bind to GLUT4myc, and to determine if the level of binding was enhanced by insulin. Following the incubation, the cells were washed and then harvested 3 days later for analysis of nNOS and HA tag expression by Western blot. As shown in Fig. 4, as little as 15 min incubation was sufficient to produce nNOS and HA expression, and protein levels after incubation for 30 and 40 min were similar. Importantly, at each time-point, 100 nM insulin markedly increased protein expression, indicating that binding and uptake of the structure into the cell was dependent on the movement of GLUT4 from the cytosol to the membrane.

### Experiment 2

There were two primary aims for the second experiment. The first aim was to test the effect of different amounts of plasmid DNA on uptake and expression. The second aim was to determine the optimal amounts of DNA relative to liposomes, as determined by the ratio of DC-Chol to DNA (w/w). As shown in Fig. 5, of all DNA concentrations, a DC-Chol to DNA ratio of 2.0 produced the highest expression of nNOS, in agreement with prior studies. In terms of DNA, 5 µg was sufficient to produce strong nNOS expression, while levels decreased at 20 µg, particularly for the higher DC-Chol to DNA ratios. Therefore, in future cell culture experiments between 5 and 10 µg of DNA were used at a DC-Chol to DNA ratio of 2.0.

### Experiment 3

In this experiment, L6-GLUT4myc myoblasts were incubated with the same structure shown in Fig. 3, except here the NOS-M plasmid was replaced with a full-length Dystrophin plasmid. This construct is driven by the ubiquitous CAG promoter and is ~20 Kb in length (Farruggio AP, et al. (2017), Biotechnol J 12(4), 1600477). The two primary aims of this experiment were (1) to test whether the very large dystrophin gene could be taken up in a targeted muscle cell and expressed using this targeting platform, and (2) to test the effects of a wide-range of insulin concentrations on the targeting and transduction of expression in muscle cells. The range of insulin begins with low levels normally found in human blood. In this experiment, cells were incubated with the vector for 15 min, washed and then harvested 3 days later. As shown in Fig. 6, full-length dystrophin was strongly expressed and dystrophin levels were greater in cells treated with insulin. Importantly, at low insulin concentrations normally found in human blood (0.5 and 1.0 nM), dystrophin expression increased compared to cells without insulin. These results support the hypothesis that enhancing or inducing GLUT4 movement to the plasma membrane by insulin leads to greater binding and uptake of the platform, and subsequent dystrophin expression.

### Experiment 4

Both rabbit polyclonal and mouse monoclonal antibodies were generated against the peptide sequence (GRQGPGGPDSI; SEQ ID NO:4), taken from the first extracellular loop of mouse GLUT4. These antibodies all show strong binding to the peptide in an ELISA assay. See, *e.g.,* Table 1, which show results of ELISA tests for 20 select mouse monoclonal antibodies.

Table 1. ELISA data showing the absorbance levels at various dilutions for 20 mouse monoclonal antibodies against the GLUT4 peptide sequence GRQGPGGPDSI (SEQ ID NO:4). All antibody cell lines strongly bind to the peptide.

| mAb Cell lines | Supernatant Dilution | | | | | | Negative Control | |
|---|---|---|---|---|---|---|---|---|
| | 1:10 | 1:30 | 1:90 | 1:270 | 1:810 | 1.2,430 | | Titer |
| 1B12-1 | 2.807 | 2.792 | 2.417 | 2.115 | 1.504 | 0.746 | 0.065 | >1:2,430 |
| 1B12-2 | 2.761 | 2.568 | 2.129 | 1.604 | 0.846 | 0.389 | 0.065 | 1:2,430 |
| 18B2-1 | 2.778 | 2.682 | 2.338 | 2.053 | 1.520 | 0.864 | 0.065 | >1:2,430 |
| 18B2-2 | 2.829 | 2.718 | 2.380 | 2.187 | 1.665 | 0.986 | 0.065 | >1:2,430 |
| 21E5-1 | 2.618 | 2.344 | 1.778 | 1.194 | 0.678 | 0.314 | 0.065 | 1:2,430 |
| 21E5-2 | 2.706 | 2.472 | 2.050 | 1.632 | 1.018 | 0.474 | 0.065 | 1:2,430 |
| 22F6-1 | 2.628 | 2.279 | 1.736 | 1.071 | 0.561 | 0.246 | 0.065 | 1:2,430 |
| 22F6-2 | 2.650 | 2.462 | 2.101 | 1.620 | 1.079 | 0.485 | 0.065 | 1:2,430 |
| 24B4-1 | 2.473 | 2.349 | 2.034 | 1.474 | 0.846 | 0.409 | 0.065 | 1:2,430 |
| 24B4-2 | 2.512 | 2.403 | 2.171 | 1.701 | 1.050 | 0.531 | 0.065 | >1:2,430 |
| 25D4-1 | 2.754 | 2.613 | 2.279 | 1.794 | 1.081 | 0.568 | 0.065 | >1:2,430 |
| 25D4-2 | 2.704 | 2.661 | 2.435 | 1.876 | 1.193 | 0.661 | 0.065 | >1:2,430 |
| 25H4-1 | 2.710 | 2.680 | 2.469 | 1.933 | 1.353 | 0.758 | 0.065 | >1:2,430 |
| 25H4-2 | 2.669 | 2.653 | 2.412 | 1.891 | 1.220 | 0.701 | 0.065 | >1:2,430 |
| 26A7-1 | 2.425 | 2.378 | 2.051 | 1.489 | 0.887 | 0.412 | 0.065 | 1:2,430 |
| 26A7-2 | 2.611 | 2.464 | 2.131 | 1536 | 0.879 | 0.438 | 0.065 | 1:2,430 |
| 29C10-1 | 2.660 | 2.453 | 2.089 | 1.661 | 0.991 | 0.468 | 0.065 | 1:2,430 |
| 29C10-2 | 2.716 | 2.482 | 2.030 | 1.530 | 0.876 | 0.417 | 0.065 | 1:2,430 |
| 30F8-1 | 2.776 | 2.708 | 2.491 | 1.967 | 1.266 | 0.699 | 0.065 | >1.2,430 |
| 30F8-2 | 2.777 | 2.696 | 2.356 | 1.802 | 1.139 | 0.590 | 0.065 | >1:2,430 |

In this experiment, a rabbit polyclonal antibody was first purified by peptide affinity purification. Next, the ability of the antibodies to bind to native GLUT4 and mediate uptake of full-length dystrophin into dystrophin-negative (*mdx*-H2K) muscle cells (Morgan *et al.,* 1994) was tested. While these cells do express some endogenous GLUT4, a determination if increasing GLUT4 expression further could improve the uptake and expression of our vector was desired. Therefore, the *mdx-*H2K cells were transfected with a plasmid containing the mouse GLUT4 gene (*mdx*-H2K-GLUT4) and, through antibacterial selection, a polyclonal cell line was established that has about two to three-fold higher GLUT4 expression. Using these dystrophin-negative cells, it was desired to assess whether increased GLUT4 expression resulted in greater binding and uptake of the platform non-viral vector, and therefore augmented dystrophin expression.

The other main aim of this experiment was to test whether albumin could enhance gene expression. The rationale for this idea is recent evidence that coating DNA/liposomal vectors with human serum albumin can enhance protein expression of the luciferase reporter in lung and spleen cells of mice. Because albumin is negatively charged at neutral pH, it can bind to the positively charged liposomes, along with the plasmid DNA. The results from this experiment reveal a number of important findings (Fig. 7). First, full-length dystrophin expression was detected and measured, indicating that the rabbit polyclonal antibodies were able to effectively bind to GLUT4 and deliver the plasmid inside muscle cells to the nucleus. Second, dystrophin expression was considerably greater in the mdx-H2K-GLUT4 cells compared to the regular *mdx-*H2K cells. Third, increasing concentrations of albumin enhanced dystrophin expression in both muscle cell lines.

### In vivo experiments

DNA/liposomal vectors for *in vivo* administration can have potential problems due to the relatively large sizes (usually ≥ 200 nm) of these structures. Therefore, a non-liposomal alternative structure was developed (Fig. 8) for enhanced *in vivo* applications. Here, the plasmid DNA is highly condensed by positively-charged Poly-L-Lysine (PLL), into structures that can have a diameter as small as 30 to 60 nm for a 20 Kb plasmid Fink TL, et al. (2006). Plasmid size up to 20 kbp does not limit effective in vivo lung gene transfer using compacted DNA nanoparticles. Gene Ther 13, 1048-1051). The PLL is biotinylated, which enables subsequent binding to NeutrAvidin. Then, the biotinylated mouse monoclonal GLUT4 antibody is added.

Finally, biotinylated recombinant mouse serum albumin is added. It is postulated that albumin promotes *in vivo* administration of this structure for a number of reasons. (1) Albumin crosses the tight endothelial barrier of muscle capillaries by transcytosis. This provides a mechanism for the movement of albumin, and therefore the disclosed non-viral vector platform, from the bloodstream to the muscle extracellular space. (2) Albumin is known to preferentially accumulate in the extracellular space of skeletal and cardiac muscle. (3) Albumin can enter muscle cells by endocytosis. This may be of particular importance in dystrophic muscles, which have increased membrane permeability and accumulate albumin intracellularly. (4) Albumin has been shown to promote endosomal escape and nuclear uptake of plasmid DNA. (5) Albumin is very stable and has a long half-life *in vivo* of 19 days. (6) Albumin is very unlikely to elicit any side-effects or immune responses, considering that it is an abundant serum protein. This will permit repeated dosing of a therapeutic payload via the non-viral vector platform.

### Experiment 5

In this first *in vivo* experiment, intramuscular (I.M.) injections were initially performed using the structure shown in Fig. 8, without the GLUT4 antibody, to determine the baseline effect of the non-viral vector platform without the targeting antibody. Two muscles (Tibialis Anterior and Gastrocnemius) were injected in one leg of 8 week old *mdx* mice, a common animal model of DMD. The structure contained the NOS-M plasmid, described in Experiment 1. Interestingly, three days later, both the Tibialis Anterior and Gastrocnemius muscles expressed NOS-M, as shown by both an nNOS antibody and an HA tag antibody (Fig. 9A-B). Presumably, the more permeable muscle fibers of the *mdx* mice allowed some uptake of the albumin-containing structure. In comparison to the injected *mdx* mice, a non-injected *mdx* control mouse had endogenous nNOS expression but did not show any HA tag expression. A non-injected WT mouse had higher endogenous nNOS expression than the *mdx* mouse but also showed no HA tag expression. Another very interesting finding was that for both muscles, but particularly for the Tibialis Anterior muscle, there was NOS-M expression (HA tag) in both the injected and non-injected legs (see Fig. 9A-B). This indicates that the structure was able to enter the systemic circulation from the injected muscles, and subsequently be taken up by the non-injected muscles of the other leg.

### Experiment 6

In the next experiment, the hypothesis that conjugating mouse monoclonal GLUT4 antibodies to the structure will greatly enhance uptake of our platform into muscles of *mdx* mice was tested. First, two mouse monoclonal antibodies were produced and purified. The correct molecular weight and purity of the antibodies was verified, under reducing and non-reducing conditions, by coomassie staining on a polyacrylamide gel (Fig. 10A). Then, the binding affinity of each antibody *in vitro* was tested using the *mdx*-H2K-GLUT4 muscle cell line. Specifically, cells were incubated with each antibody at different dilutions (from a 1 mg/ml starting concentration) for 20 min at 37°C, with or without 100 nM insulin. Cells were washed, incubated with a secondary-HRP antibody and washed again. A colorimetric assay was used to quantify the relative amount of bound antibody to the native GLUT4 protein by measuring the absorbance with a spectrophotometer. As shown in Fig. 10B, both antibodies, Clone 1 and Clone 2, bound to the native GLUT4 protein in a dose-dependent manner. At all dilutions, Clone 2 had a higher absorbance than Clone 1. Importantly, for both antibodies, insulin (100 nM) increased the absorbance (see Fig. 10B), indicating greater binding occurred when more GLUT4 was brought to the plasma membrane by insulin signaling.

Next, the Clone 2 antibody was biotinylated and incorporated into the non-viral vector platform by binding to NeutrAvidin. As a control the non-viral vector platform was also constructed without the antibody (as described in Experiment 5). Two *mdx* mice were injected I.M. with or without the antibody conjugated to the non-viral vector platform. Again, the TA and Gastrocnemius muscles were injected in one leg only. Six days after the injection, muscles were harvested and used for Western blot analysis. As shown in Fig. 11, the expression level of nNOS, as measured by the HA tag, was considerably (several fold) higher in both the TA and Gastrocnemius muscles of the mouse injected with the platform containing the Clone 1 mAb. This data indicates that the Glut4 antibody dramatically enhances the targeting and uptake of the non-viral vector platform into skeletal muscle.

### Experiment 7

Next, it was tested whether intravenous (I.V.) administration of the targeted, non-viral platform could lead to systemic delivery and expression of the proteins encoding genes of various sizes in muscle. To test this hypothesis, three genes of different sizes were used (Experiments 7-9). In this experiment, the smallest of these genes was used. This gene is α-syntrophin, with a plasmid size of ~5.5 kb. Here, *mdx4CV* mice were injected I.M. or I.V. with the non-viral platform described in Fig. 11, containing the α-syntrophin gene, which has a RAS palmitoylation sequence at the C-terminal for targeting to the sarcolemma. Targeting of α-syntrophin to the sarcolemma in normal muscle occurs by interaction with dystrophin, which is absent in *mdx4*CV muscle. As shown in Fig. 12, both I.M. and I.V. delivery resulted in a considerable increase of α-syntrophin expression in muscle 12 days after injection, compared to a non-injected control mouse, both confirmed by Western blot (Fig. 12A) and Immunostaining (Fig. 12B) of muscle cross-sections.

### Experiment 8

The second gene was nNOS, as described in Fig. 11, with a plasmid size of ~10.5 kb. Again *mdx4CV* mice were injected either I.M. or I.V. Specifically, two mice were injected I.M. in the gastrocnemius muscle of one hindlimb and two mice were injected I.V. via the retro-orbital sinus. As shown in Fig. 13A, nNOS expression was considerably higher (~50%) in muscles of mice after I.M. and I.V. injections compared to an untreated control muscle. Utrophin (a homologue of dystrophin) expression was also measured here by Western blot where it was shown to increase considerably in mice injected with the nNOS construct (see Fig. 13A). In another experiment, the effect of nNOS plasmid dose was investigated. Here, *mdx* mice were injected I.V. with the platform containing different doses of nNOS plasmid (20, 60 or 120 µg). As shown in Fig. 13B, there was a progressive increase in nNOS expression in the TA muscle as the nNOS plasmid dose was increased, as measured both by nNOS and HA tag expression.

### Experiment 9

The third and largest gene tested was full-length mouse dystrophin. Here, two plasmid constructs were used, each containing full-length dystrophin but with different promoters and plasmid backbones. The first construct contained the truncated muscle-specific creatine kinase 8 promoter (CK8) (PMID: 21266958, PMID: 19298131). This construct was 15.5 kb. The second construct contained the ubiquitous *cytomegalovirus* (CMV) promoter. This construct was 17.5 kb. As shown in Fig. 14, *mdx*4CV mice injected I.V. with the CK8 construct showed expression of full-length dystrophin in a number of hindlimb muscles. The levels of expression were much higher than a non-injected *mdx*4CV mouse, which had faint bands on the Western blot due to the presence of a very small number of revertant fibers that express dystrophin (PMID: 1635838). Also, in this experiment we used a membrane-enriched muscle lysate for Western blotting, which likely enables even very low levels of skeletal muscle dystrophin in revertant fibers to be detected. Comparison with a WT mouse showed dystrophin levels in the injected mice ranging from ~0.2 to 0.5% for different muscles. Next, we injected *mdx4CV* mice 1.V. with the platform containing either the CK8 or the CMV construct to measure dystrophin expression in the heart. Importantly, as shown in Fig. 15, cardiac muscles of injected mice showed clear expression of full-length dystrophin compared with a non-injected control. The level of dystrophin expression was higher with the CMV construct compared to CK8, with values ~ 0.1 to 0.2% of a WT mouse. Together, the data from Experiments 8 and 9 indicate that the non-viral platform composition (see Fig. 8) containing a large gene such as full-length dystrophin is effectively taken up and expressed by both skeletal and cardiac muscle.

### Experiment 10

In this experiment, particle tracking analysis was performed in order to determine the size distribution of the particles produced using our non-viral platform. Here, the platform composition (see Fig. 8) contained the CK8-full-length dystrophin plasmid. Particle tracking analysis was performed using the standard procedures for the NanoSight ns300 (Malvern Panalytical), which allows reliable, rapid measurement of nanoparticle size distribution of particles ranging in diameter from 0.01 to 1.0 µm. The platform sample was diluted 1:100 with deionized water and then 4 separate readings were carried out of the particle size distribution. As shown in Fig. 20, the particle size distribution plot indicates that the modal (peak) particle size was 171 nm. Overall, the mean particle size was 236 nm ± 4.4 and. Data analysis illustrated that 50% of the particles were less than 213 nm, and 90% of particles were less than 344 nm. The mean size of our particles is very similar to the mean size of particles containing NeutrAvidin and siRNA (237 nm), which were effective at *in vivo* gene silencing in mice (PMID: 28026957). Therefore, the data presented in this experiment indicates that even with a large plasmid DNA such as full-length dystrophin (15.5 kb), the non-viral platform forms small particles that can be efficiently taken up and expressed by muscle in vivo (see Figs. 14 and 15).

### Experiment 11

In this experiment, a number of monoclonal antibodies against GLUT 4 were produced and the binding affinity of each antibody was evaluated. Mouse monoclonal antibodies against both the mouse GLUT4 peptide (GRQGPGGPDSI; SEQ ID NO:4) and human GLUT4 peptide (GRQGPEGPSSI; SEQ ID NO:2.) were manufactured by Genscript (NJ, USA). A total of 14 unique clones were generated; 6 against the mouse GLUT4 sequence and 8 against the human GLUT4 sequence. Each hybridoma cell line was grown in cell culture for several weeks and monoclonal antibodies were purified using protein A/G latex beads. The binding affinity of each monoclonal antibody was measured by ELISA. For the mouse GLUT4 sequence antibodies, the ELISA plate was coated with the peptide (SEQ ID NO:4). For the human GLUT4 sequence antibodies, recombinant human GLUT4 protein was used to coat the ELISA plate. In both cases, each well of the ELISA plate was coated with 100 ng of antigen overnight at 4°C. After blocking, the monoclonal antibodies were added for 1 hr at RT. After washing, secondary mouse-HRP antibody was added for 1 hr at RT. After further washing, a chromogenic substrate for HRP (1-STEP^{™} Ultra TMB-ELISA substrate solution; Thermo Fisher Scientific) was added for 10 to 20 min. Finally, the reaction was stopped by adding 2M sulfuric acid and the absorbance at 450 nm was measured on a plate reader. As shown in Fig. 16, all 6 monoclonal antibodies displayed a concentration-dependent increase in absorbance. The best binding clones were 25D4 followed by 22F6 and 21E5. The other three clones showed similar, lower binding curves (see Fig. 16). For the human GLUT for sequence monoclonal antibodies, the same method was used, except the plate was coated with recombinant human GLUT4 protein. As shown in Fig. 17, 7 monoclonal antibodies displayed a dose-dependent increase in absorbance, with clones 5E3 and 8G2 having the best binding affinity and clones 2B9 and 4G2 showing the weakest binding affinity. One clone, 15G2, was not able to be assayed due to insufficient antibody production by this hybridoma cell line.

In order to test whether the mouse sequence antibodies could bind to the human GLUT4 protein or whether the human sequence antibodies could bind to the mouse GLUT4 peptide sequence, two additional ELISA experiments were carried out. As shown in Fig. 18, two antibodies that bound strongly to the mouse sequence (22F6 and 21E5) did not bind effectively to the human GLUT4 protein, while antibodies made against the human GLUT4 peptide sequence (5E3 and 8G2) showed strong binding. As shown in Fig. 19, in the reverse experiment, antibodies made against the human GLUT4 sequence bound with varying degrees of affinity to the mouse GLUT4 peptide (8G2 was the best while 12E11 did not bind) but all four of these antibodies bound much less strongly than antibodies made against the mouse sequence (22F6 and 21E5). Therefore, despite 83.3% homology between the mouse and human peptide sequences, the antibodies produced show much stronger binding to the species-specific GLUT4 sequence against which they were derived.

### Experiment 12

For each of the 14 unique monoclonal antibodies generated; 6 against the mouse GLUT4 sequence and 8 against the human GLUT4 sequence, the heavy and light chain variable regions were sequenced by GenScript (NJ, USA) using the following method. Total RNA was isolated from the hybridoma cells (TRIzol^{®} Reagent). Total RNA was then reverse-transcribed into cDNA using either isotype-specific anti-sense primers or universal primers (PrimeScript^{™} 1st Strand cDNA Synthesis Kit). Antibody fragments of heavy chain and light chain were amplified according to the standard operating procedure (SOP) of rapid amplification of cDNA ends (RACE) of GenScript. Amplified antibody fragments were cloned into a standard cloning vector separately. Colony PCR was performed to screen for clones with inserts of correct sizes and the sequences of each antibody were provided. In Table 2, the Complementarity Determining Regions (CDRs) for the Heavy and Light chains are shown for the 6 monoclonal antibodies produced against the mouse GLUT 4 peptide (GRQGPGGPDSI; SEQ ID NO:4). Predicted consensus sequences are also shown for each CDR. In Table 3, the Complementarity Determining Regions (CDRs) for the Heavy and Light chains are shown for the 8 monoclonal antibodies produced against the human GLUT 4 peptide (GRQGPEGPSSI; SEQ ID NO:2.). Predicted consensus sequences are also shown for each CDR. Interestingly, two clones (2B9 and 4G7) that bound most weakly to the human GLUT protein in the ELISA assay (see Fig. 17) did not contain the arginine (R) found in the CDR3_{VH} consensus sequence of the other 6 clones, suggesting that this reside may have an important role in antigen binding activity. Note also that clone 15G2 was not represented in the ELISA assay (see Fig. 17) since this antibody did not grow well under our culture conditions.

Table 2 shows the results from Experiment 12, listing the amino acid sequences of the Complementarity Determining Regions (CDRs) for the Heavy Chain (V_{H}) and Light Chain (V_{L}) of 6 mouse monoclonal antibody clones against the mouse GLUT4 peptide sequence GRQGPGGPDSI (SEQ ID NO:4). Also shown below Table 2 is an illustrative consensus sequence for each CDR.

| **Clone(s)** | **CDR1 (V_{H})** | **CDR2 (V_{H})** | **CDR3 (V_{H})** |
|---|---|---|---|
| 25D4 | TYGMS (SEQ ID NO:58) | WINTSSGVPTYADDFKG (SEQ ID NO:60) | PIHMVVAEDY (SEQ ID NO:66) |
| 24H4 | TYGMT (SEQ ID NO:59) | WINTYSGVPTYANDFKG (SEQ ID NO:61) | PIHKVVAEDY (SEQ ID NO:67) |
| 22F6 | TYGMS (SEQ ID NO:58) | WINTYSGLPTYTNDFKG (SEQ ID NO:62) | PITTVVPFDY (SEQ ID NO:68) |
| 21E5 | TYGMS (SEQ ID NO:58) | WINTYSGLPTYADDFKG (SEQ ID NO:63) | PITTVVPFDY (SEQ ID NO:69) |
| 18B2 | TYGMS (SEQ ID NO:58) | WINTYSGVPTYADDFKG (SEQ ID NO:64) | PITKVVAGDF (SEQ ID NO:70) |
| 30F8 | TYGMS (SEQ ID NO:58) | WINTFSGVPTYTDDFKG (SEQ ID NO:65) | PITTVVPFDY (SEQ ID NO:71) |

| **Clone(s)** | **CDR1 (V_{L})** | **CDR2 (V_{L})** | **CDR3 (V_{L})** |
|---|---|---|---|
| 25D4 | KSSQILLYGRNQKNYLA (SEQ ID NO:72) | WASTRES (SEQ ID NO:78) | QQYYSKPYT (SEQ ID NO:84) |
| 24H4 | RSSQSLLYSGNQKNYLA (SEQ ID NO:73) | WASTWES (SEQ ID NO:79) | QQYYTFPYT (SEQ ID NO:85) |
| 22F6 | RSSQSLVYTYGNTYLH (SEQ ID NO:74) | KVSNRFS (SEQ ID NO:80) | SQSRHSPWT (SEQ ID NO:86) |
| 21E5 | RSSQSLVYNYGNTYLH (SEQ ID NO:75) | KVSNRFS (SEQ ID NO:81) | SQSRHSPWT (SEQ ID NO:87) |
| 18B2 | KSSQSLLYRSNQKNYLA (SEQ ID NO:76) | WASTRES (SEQ ID NO:82) | QQYFSSPYT (SEQ ID NO:88) |
| 30F8 | RSSQSLVYSTGNTYLH (SEQ ID NO:77) | KVSNRFS (SEQ ID NO:83) | SQSRHGPWT (SEQ ID NO:89) |

### CDR consensus sequences

CDR1 (V_{H}): T Y G M S/T
CDR2 (V_{H}): W I N T Y/F/S S G L/V P T Y X X D F K G
CDR3 (V_{H}): P I H/T X V V A/P X D Y
CDR1 (V_{L}): K/R S S Q S L L/V Y X₅₋₆ Y L A/H
CDR2 (V_{L}): K/W A/V S N/T R E/F S
CDR3 (V_{L}): Q Q S/Y F/R/Y H/S/T X P Y/W T

Table 3 shows the results from Experiment 12, listing amino acid sequences of the Complementarity Determining Regions (CDRs) for the Heavy Chain (V_{H}) and Light Chain (V_{L}) of 8 monoclonal antibody clones against the human GLUT4 peptide sequence GRQGPEGPSSI (SEQ ID NO:2). Also shown below Table 3 is an illustrative consensus sequences for each CDR.

| **Clone** | **CDR1(V_{H})** | **CDR2(V_{H})** | **CDR3(V_{H})** |
|---|---|---|---|
| 2B9 | DDAMH (SEQ ID NO:10) | WINTETGEPTYADDFKG (SEQ ID NO:18) | LDF (contained in SEQ ID NO:26) |
| 4G7 | DYSMH (SEQ ID NO:11) | WINTETGEPTYADDFKG (SEQ ID NO:19) | GASGY (SEQ ID NO:27) |
| 5E3 | EYTIH (SEQ ID NO:12) | GINCNSGGTSYTQKFKD (SEQ ID NO:20) | RFFYGSSQFAY (SEQ ID NO:28) |
| 15G2 | DYSMH (SEQ ID NO:13) | WINTETGEPTYADDFKG( SEQ ID NO:21) | RGEF (SEQ ID NO:29) |
| 8G2 | DFSMH (SEQ ID NO:14) | WINTETGDPTYADDFKG (SEQ ID NO:22) | RGDY (SEQ ID NO:30) |
| 1A7 | AYSMH (SEQ ID NO:15) | WINTETGEATYADDFKG (SEQ ID NO:23) | RGDY (SEQ ID NO:31) |
| 6D1 | DYSMH (SEQ ID NO:16) | WINTETGEPTYADDFKG (SEQ ID NO:24) | RGDY (SEQ ID NO:32) |
| 12E11 | DYSMH (SEQ ID NO:17) | WINTETGEPTYADDFKG (SEQ ID NO:25) | GRSAY (SEQ ID NO:33) |

| **Clone** | **CDR1(V_{L})** | **CDR2(V_{L})** | **CDR3(V_{L})** |
|---|---|---|---|
| 2B9 | RSSQSLVHSNGKTYLH (SEQ ID NO:34) | KVSNRFS (SEQ ID NO:42) | SQSTHVPWT (SEQ ID NO:50) |
| 4G7 | KASQSVSKNVA (SEQ ID NO:35) | SSSKRYS (SEQ ID NO:43) | HQDYNSPWT (SEQ ID NO:51) |
| 5E3 | RASKSVTTSGYSYMH (SEQ ID NO:36) | LASNLES (SEQ ID NO:44) | QHSRELPLT (SEQ ID NO:52) |
| 15G2 | KASQSVNKNVA (SEQ ID NO:37) | SASKRYT (SEQ ID NO:45) | LQDYTSPWT (SEQ ID NO:53) |
| 8G2 | RSSQSLVHSNRNIYLH (SEQ ID NO:38) | KVSNRFS (SEQ ID NO:46) | SQDKYIPWT (SEQ ID NO:54) |
| 1A7 | RSSQSLVRSNRNIYLH (SEQ ID NO:39) | KVSNRFS (SEQ ID NO:47) | SQDSHIPWT (SEQ ID NO:55) |
| 6D1 | RSSQSLVHNNRNIYLH (SEQ ID NO:40) | KVSNRFS (SEQ ID NO:48) | SQDRYIPWT (SEQ ID NO:56) |
| 12E11 | KASQTVSNNVA (SEQ ID NO:41) | SASNRYT (SEQ ID NO:49) | QQDYSSPWT (SEQ ID NO:57) |

### CDR consensus sequences

CDR1 (V_{H}): D/E Y SIT I/M H
CDR2 (V_{II}): W I N T E S/T G D/E X T/S Y A D D F K G
CDR3 (V_{H}): R X X Y
CDR1 (V_{L}): R/K A/S S Q S L/V X X N/S
CDR2 (V_{L}): V/A S N R F/Y S/T
CDR3 (V_{L}): Q D X X X P W T

### Conclusion

It is demonstrated that the disclosed non-viral vector platform can efficiently and specifically deliver therapeutic payloads to tissues that express GLUT4, including but not limited to skeletal muscle, cardiac muscle, adipose tissue, smooth muscle, certain areas of the brain (*e.g.* hippocampus and cerebellum) and certain cancer cells (*e.g.* melanocytes). Therapeutics that can be delivered to these tissues by our platform include nucleic acids (e.g., plasmid DNA, minicircle DNA, mRNA, siRNA, shRNA, oligonucleotides, CRISPR/Cas9 constructs) proteins, peptides, lipids and drugs. The platform is not limited to payloads of particular sizes, but rather can successfully deliver vary large nucleic acid constructs resulting in the uptake and expression of the encoded large transgenes. The effects and efficiencies of the targeting and uptake are augmented further by insulin induction of GLUT4 translocation to the cell's plasma membrane and by incorporation of albumin to the platform vector composition. Therapeutic compositions incorporating the disclosed non-viral vector platform can be delivered as a monotherapy or as part of a combination therapy.

While illustrative embodiments have been illustrated and described, it will be appreciated that various changes can be made therein without departing from the spirit and scope of the invention.

### CLAUSES:

1. A composition comprising an antibody, or fragment or derivative thereof, that specifically binds to glucose transporter 4 ("GLUT4") protein, and a therapeutic payload conjugated to the antibody, or fragment or derivative thereof.
2. The composition of Clause 1, wherein the antibody, or fragment or derivative thereof, specifically binds to a first extracellular loop domain of GLUT4.
3. The composition of Clause 2, wherein the first extracellular loop domain of GLUT4 comprises an amino acid sequence with at least 80% identity to the sequence INAPQKVIEQSYNETWLGRQGPEGPSSIPPGTLTTL(SEQ ID NO: 1).
4. The composition of Clause 2, wherein the first extracellular loop domain of GLUT4 comprises an amino acid sequence with at least 80% identity to the sequence GRQGPEGPSSI (SEQ ID NO:2).
5. The composition of Clause 2, wherein the antibody, or fragment or derivative thereof, specifically binds to a subdomain of the first extracellular loop domain of GLUT4 that comprises an amino acid sequence with at least 80% identity to the sequence GRQGPGGPDSI(SEQ ID NO:4).
6. The composition of Clause 2, wherein the antibody, or fragment or derivative thereof, specifically binds to a subdomain of the first extracellular loop domain of GLUT4 that comprises a sequence with at least 80% identity to the sequence GRQGPEGPSSI (SEQ ID NO:2).
7. The composition of Clause 1, wherein the antibody is a monoclonal antibody.
8. The composition of Clause 1, wherein the antibody fragment or antibody derivative is or comprises a single chain antibody, an Fab fragment, an F(ab)2 fragment, a V_{H}H fragment, a V_{NAR} fragment, or a nanobody.
9. The composition of Clause 8, wherein the single-chain antibody is a single chain variable fragment (scFv), or a single-chain Fab fragment (scFab).
10. The composition of Clause 1, wherein the antibody, or fragment or derivative thereof, is biotinylated.
11. The composition of Clause 1, wherein the therapeutic payload comprises a nucleic acid, a protein or peptide, a lipid, a small molecule pharmaceutical, or a radioisotope.
12. The composition of Clause 11, wherein the therapeutic payload comprises a nucleic acid, wherein the nucleic acid is selected from DNA, mRNA, siRNA, and shRNA.
13. The composition of Clause 11, wherein the therapeutic payload comprises a nucleic acid, wherein the nucleic acid comprises an open reading frame operatively linked to a promoter sequence.
14. The composition of Clause 11, wherein the therapeutic payload comprises a nucleic acid in linear form, a nucleic acid in plasmid form, or a nucleic acid in minicircle form.
15. The composition of Clause 14, further comprising a histone protein or a plurality of histone proteins associated with the nucleic acid.
16. The composition of Clause 15, wherein the histone protein or at least a portion of the plurality of histone proteins is biotinylated.
17. The composition of Clause 14, further comprising a liposome associated with the nucleic acid.
18. The composition of Clause 17, wherein the liposome comprises one or more lipids selected from DC-Cholesterol-HCl,3b-[N-(N',N'-dimethylaminoethane)-caramoyl]cholesterol hydrochloride ("DC-Chol"), 1,2-dioleoyl-sn-glycero-3-phosphoethanolamine ("DOPE"), 1,2-di-O-octadecenyl-3-trimethylammonium ("DOTMA"), N-[1-(2,3-Dioleoyloxy)propyl]-N,N,N-trimethylammonium propane ("DOTAP"), dimethyldioctadecylammonium bromide ("DDAB"), 1,2-dimyristoyl-3-trimethylammonium-propane ("DMTAP"), 1,2-dioleoyl-3-dimethylammonium-propane ("DODAP"), cholesteryl hemisuccinate ("CHEMS") and cholesterol ("CHOL"), 2,3-dioleyloxy-N-[2(sperminecarboxamido)ethyl]-N,N-dimethyl-1-propanaminium ("DOSPA"), Di-octadecyl-amido-glycyl-spermine ("DOGS"), dioleoylphosphatidylcholine ("DOPC"), and the like.
19. The composition of Clause 17, wherein the liposome further comprises polyethylene glycol ("PEG").
20. The composition of Clause 14, further comprising a plurality of cationic peptide moieties associated with the nucleic acid, thereby resulting in condensed nucleic acid.
21. The composition of Clause 20, wherein the cationic peptide moieties are or comprise poly-L-Lysine ("PLL"), linear *Polyethylenimine* ("LPEI"), branched *Polyethylenimine* ("BPEI"), chitosan, spermidine and spermine, Polyamidoamine ("PAMAM"), poly(2-dimethylaminoethyl methacrylate) (*"PDMAEMA"*)*,* Poly(beta-amino ester)s (*"PBAEs"*)*,* poly {*N*-[N-(2-aminoethyl)-2-aminoethyl]aspartamide ("PAsp(DET)"), poly(2-aminoethyl ethylene phosphate ("PPEEA"), and the like.
22. The composition of Clause 20, wherein at least a portion of the plurality the cationic peptide moieties is biotinylated.
23. The composition of Clause 1, further comprising albumin.
24. The composition of Clause 23, wherein at least a portion of the albumin is biotinylated.
25. The composition of any one of Clauses 16, 22, and 24, wherein the antibody, or fragment or derivative thereof, is biotinylated and the composition further comprises neutravidin.
26. The composition of Clause 11, wherein the therapeutic payload comprises a nucleic acid with a promoter sequence operatively linked to a sequence encoding a dystrophin protein, or a functional fragment thereof.
27. The composition of Clause 26, wherein the dystrophin protein is a substantially full-length human dystrophin protein.
28. The composition of Clause 26, wherein the full-length human dystrophin protein has an amino acid sequence with at least 80% sequence identity to the sequence set forth in SEQ ID NO:5.
29. A method of targeting a therapeutic payload for delivery to a cell that expresses GLUT4, the method comprising contacting the cell with the composition as recited in any one of Clauses 1-28.
30. The method of Clause 29, further comprising contacting the cell with insulin prior to contacting the cell with the composition.
31. The method of Clause 29, wherein the cell is a skeletal muscle cell, a cardiac muscle cell, a smooth muscle cell, an adipose tissue cell, a hippocampal cell, or a cerebellum cell.
32. The method of Clause 29, wherein the cell is a melanoma cell, prostate cancer cell, cancer cell of muscle tissue, such as rhabdomyosarcoma cell, or a breast cancer cell.
33. A method of treating a disease or condition in a subject with a genetic mutation in a gene encoding dystrophin protein, comprising administering to a subject a therapeutically effective amount of a composition comprising:
   an antibody, or fragment or derivative thereof, that specifically binds glucose transporter 4 ("GLUT4") protein, and
   a therapeutic payload conjugated to the antibody, or fragment or derivative thereof, wherein the therapeutic payload comprises a nucleic acid with a promoter sequence operatively linked to a sequence encoding a dystrophin protein, or a functional fragment thereof.
34. The method of Clause 33, wherein the subject has or is a genetic carrier of Duchenne muscular dystrophy ("DMD") or Becker muscular dystrophy ("BMD").
35. The method of Clause 33, wherein the antibody, or fragment or derivative thereof, specifically binds to a first extracellular loop domain of GLUT4.
36. The method of Clause 35, wherein the first extracellular loop domain of GLUT4 comprises an amino acid sequence with at least 80% identity to the sequence INAPQKVIEQSYNETWLGRQGPEGPSSIPPGTLTTL (SEQ ID NO: 1).
37. The method of Clause 35, wherein the antibody, or fragment or derivative thereof, specifically binds to a subdomain of the first extracellular loop domain of GLUT4 that comprises a sequence with at least 80% identity to the sequence GRQGPEGPSSI (SEQ ID NO:2).
38. The method of Clause 33, wherein the antibody is a monoclonal antibody.
39. The method of Clause 33, wherein the antibody fragment or antibody derivative is or comprises a single chain antibody, an Fab fragment, an F(ab)2 fragment, a V_{H}H fragment, a V_{NAR} fragment, or a nanobody.
40. The method of Clause 39, wherein the single-chain antibody is a single chain variable fragment (scFv), or a single-chain Fab fragment (scFab).
41. The method of Clause 33, wherein the antibody, or fragment or derivative thereof, is biotinylated.
42. The method of Clause 33, wherein the nucleic acid is in linear form, plasmid form, or minicircle form.
43. The method of Clause 42, wherein the composition further comprises a histone protein or a plurality of histone proteins associated with the nucleic acid.
44. The method of Clause 43, wherein the histone protein or at least a portion of the plurality of histone proteins is biotinylated.
45. The method of Clause 39, wherein the composition further comprises a liposome associated with the nucleic acid.
46. The method of Clause 45, wherein the liposome comprises one or more lipids selected from DC-Cholesterol-HCl,3b-[N-(N',N'-dimethylaminoethane)-caramoyl]cholesterol hydrochloride ("DC-Chol"), 1,2-dioleoyl-sn-glycero-3-phosphoethanolamine ("DOPE"), 1,2-di-O-octadecenyl-3-trimethylammonium ("DOTMA"), N-[1-(2,3-Dioleoyloxy)propyl]-N,N,N-trimethylammonium propane ("DOTAP"), dimethyldioctadecylammonium bromide ("DDAB"), 1,2-dimyristoyl-3-trimethylammonium-propane ("DMTAP"), 1,2-dioleoyl-3-dimethylammonium-propane ("DODAP"), cholesteryl hemisuccinate ("CHEMS") and cholesterol ("CHOL"), 2,3-dioleyloxy-N-[2(sperminecarboxamido)ethyl]-N,N-dimethyl-1-propanaminium ("DOSPA"), Di-octadecyl-amido-glycyl-spermine ("DOGS"), dioleoylphosphatidylcholine ("DOPC"), and the like.
47. The method of Clause 45, wherein the liposome further comprises polyethylene glycol ("PEG").
48. The method of Clause 33, further comprising a plurality of cationic peptide moieties associated with the nucleic acid, thereby resulting in condensed nucleic acid.
49. The method of Clause 48, wherein the cationic peptide moieties are or comprise poly-L-Lysine ("PLL"), linear *Polyethylenimine ("LPEI"), branched Polyethylenimine* ("BPEI"), chitosan, spermidine and spermine, Polyamidoamine ("PAMAM"), poly(2-dimethylaminoethyl methacrylate) (*"PDMAEMA"*), Poly(beta-amino ester)s (*"PBAEs*")*,* poly {*N*-[*N*-(2-aminoethyl)-2-aminoethyl]aspartamide ("PAsp(DET)"), poly(2-aminoethyl ethylene phosphate ("PPEEA"), and the like.
50. The method of Clause 48, wherein at least a portion of the plurality the cationic peptide moieties is biotinylated.
51. The method of Clause 33, wherein the composition further comprises albumin.
52. The method of Clause 46, wherein at least a portion of the albumin is biotinylated.
53. The method of any one of Clauses 44, 50, and 52, wherein the antibody, or fragment or derivative thereof, is biotinylated and the composition further comprises neutravidin.
54. The method of Clause 33, wherein the encoded dystrophin protein is a substantially full-length human dystrophin protein.
55. The method of Clause 33, wherein the full-length human dystrophin protein has an amino acid sequence with at least 80% sequence identity to the sequence (SEQ ID NO:5).
56. The method of Clause 33, wherein the subject is a human.
57. An anti-GLUT4 antibody, or fragment or derivative thereof comprising a heavy chain variable region (V_{H}) and a light chain variable region (V_{L}), wherein:
   a) the V_{H} comprises a CDR1 comprising a sequence (D/E)Y(S/T)(I/M)H, a CDR2 comprising a sequence WINTE(S/T)G(D/E)X₁(T/S)YADDFKG, and a CDR3 comprising a sequence RX₂X₃Y; and
   b) the V_{L} comprises a CDR1 comprising a sequence (R/K)(A/S)SQS(L/V)X₄X₅ (N/S), a CDR2 comprising a sequence (V/A)SNR(F/Y)(S/T), and a CDR3 comprising a sequence QDX₆ X₇X₈P T.
58. The anti-GLUT4 antibody of Clause 57, wherein X₁ is P or T; X₂, is A, F, S, or G; X₃ is A, D, E, or G; X₄ is S, V, N, or T; X₅ is N, H, R, K, or T; X₆ is R, Y, S, T, or K; X₇ is H N, E, T, Y, or S; or X₈ is V, S, L or I.
59. An anti-GLUT4 antibody, or fragment or derivative thereof comprising a heavy chain variable region (V_{H}) and a light chain variable region (V_{L}), wherein:
   a) the V_{H} comprises a VH-CDR1 having a sequence selected from SEQ ID NOs: 10-17, a VH-CDR2 having a sequence selected from SEQ ID NOs:18-25, and a VH-CDR3 having a sequence selected from LDF and SEQ ID NOs:27-33; and
   b) the V_{L} comprises a VL-CDR1 having a sequence selected from SEQ ID NOs:34-41, a VL-CDR2 having a sequence selected from SEQ ID NOs:42-49, and a VL-CDR3 having a sequence selected from SEQ ID NOs:50-57.
60. An anti-GLUT4 antibody of Clause 59, wherein:
   a) the V_{H} comprises a VH-CDR1 having a sequence set forth in SEQ ID NO 10, a VH-CDR2 having a sequence set forth in SEQ ID NO:18, and a VH-CDR3 having a sequence LDF; and the V_{L} comprises a VL-CDR1 having a sequence set forth in SEQ ID NO:34, a VL-CDR2 having a sequence set forth in SEQ ID NO:42, and a VL-CDR3 having a sequence set forth in SEQ ID NO:50;
   b) the V_{H} comprises a VH-CDR1 having a sequence set forth in SEQ ID NO:11, a VH-CDR2 having a sequence set forth in SEQ ID NO:19, and a VH-CDR3 having a sequence set forth in SEQ ID NO:27; and the V_{L} comprises a VL-CDR1 having a sequence set forth in SEQ ID NO:35, a VL-CDR2 having a sequence set forth in SEQ ID NO:43, and a VL-CDR3 having a sequence set forth in SEQ ID NO:51;
   c) the V_{H} comprises a VH-CDR1 having a sequence set forth in SEQ ID NO:12, a VH-CDR2 having a sequence set forth in SEQ ID NO:20, and a VH-CDR3 having a sequence set forth in SEQ ID NO:28; and the V_{L} comprises a VL-CDR1 having a sequence set forth in SEQ ID NO:36, a VL-CDR2 having a sequence set forth in SEQ ID NO:44, and a VL-CDR3 having a sequence set forth in SEQ ID NO:52;
   d) the V_{H} comprises a VH-CDR1 having a sequence set forth in SEQ ID NO 13, a VH-CDR2 having a sequence set forth in SEQ ID NO:21, and a VH-CDR3 having a sequence set forth in SEQ ID NO:29; and the V_{L} comprises a VL-CDR1 having a sequence set forth in SEQ ID NO:37, a VL-CDR2 having a sequence set forth in SEQ ID NO:45, and a VL-CDR3 having a sequence set forth in SEQ ID NO:53;
   e) the V_{H} comprises a VH-CDR1 having a sequence set forth in SEQ ID NO:14, a VH-CDR2 having a sequence set forth in SEQ ID NO:22, and a VH-CDR3 having a sequence set forth in SEQ ID NO:30; and the V_{L} comprises a VL-CDR1 having a sequence set forth in SEQ ID NO:38, a VL-CDR2 having a sequence set forth in SEQ ID NO:46, and a VL-CDR3 having a sequence set forth in SEQ ID NO:54;
   f) the V_{H} comprises a VH-CDR1 having a sequence set forth in SEQ ID NO: 15, a VH-CDR2 having a sequence set forth in SEQ ID NO:23, and a VH-CDR3 having a sequence set forth in SEQ ID NO:31; and the V_{L} comprises a VL-CDR1 having a sequence set forth in SEQ ID NO:39, a VL-CDR2 having a sequence set forth in SEQ ID NO:47, and a VL-CDR3 having a sequence set forth in SEQ ID NO:55;
   g) the V_{H} comprises a VH-CDR1 having a sequence set forth in SEQ ID NO:16, a VH-CDR2 having a sequence set forth in SEQ ID NO:24, and a VH-CDR3 having a sequence set forth in SEQ ID NO:32; and the V_{L} comprises a VL-CDR1 having a sequence set forth in SEQ ID NO:40, a VL-CDR2 having a sequence set forth in SEQ ID NO:48, and a VL-CDR3 having a sequence set forth in SEQ ID NO:56; or
   h) the V_{H} comprises a VH-CDR1 having a sequence set forth in SEQ ID NO:17, a VH-CDR2 having a sequence set forth in SEQ ID NO:25, and a VH-CDR3 having a sequence set forth in SEQ ID NO:33; and the V_{L} comprises a VL-CDR1 having a sequence set forth in SEQ ID NO:41, a VL-CDR2 having a sequence set forth in SEQ ID NO:49, and a VL-CDR3 having a sequence set forth in SEQ ID NO:57.
61. A composition comprising an antibody, or fragment or derivative thereof, of any one of Clauses 57-60, and a therapeutic payload conjugated to the antibody, or fragment or derivative thereof.
62. The composition of Clause 61, wherein the antibody fragment or antibody derivative is or comprises a single chain antibody, an Fab fragment, an F(ab)2 fragment, a V_{H}H fragment, a V_{NAR} fragment, or a nanobody.
63. The composition of Clause 62, wherein the single-chain antibody is a single chain variable fragment (scFv), or a single-chain Fab fragment (scFab).
64. The composition of Clause 61, wherein the therapeutic payload comprises a nucleic acid, a protein or peptide, a lipid, a small molecule pharmaceutical, or a radioisotope.
65. The composition of Clause 64, wherein the therapeutic payload comprises a nucleic acid, wherein the nucleic acid is selected from DNA, mRNA, siRNA, and shRNA.
66. The composition of Clause 64, wherein the therapeutic payload comprises a nucleic acid, wherein the nucleic acid comprises an open reading frame operatively linked to a promoter sequence.
67. The composition of Clause 64, wherein the therapeutic payload comprises a nucleic acid in linear form, a nucleic acid in plasmid form, or a nucleic acid in minicircle form.
68. The composition of Clause 64, further comprising a histone protein or a plurality of histone proteins associated with the nucleic acid.
69. The composition of Clause 67, further comprising a liposome associated with the nucleic acid.
70. The composition of Clause 67, further comprising a plurality of cationic peptide moieties associated with the nucleic acid, thereby resulting in condensed nucleic acid.
71. The composition of Clause 61, wherein the therapeutic payload comprises a nucleic acid with a promoter sequence operatively linked to a sequence encoding a dystrophin protein, or a functional fragment thereof.
72. The composition of Clause 71, wherein the dystrophin protein is a substantially full-length human dystrophin protein having an amino acid sequence with at least 80% sequence identity to the sequence set forth in SEQ ID NO:5.
73. A method of targeting a therapeutic payload for delivery to a cell that expresses GLUT4, the method comprising contacting the cell with the composition as recited in any one of Clauses 61-72.
74. The method of Clause 73, further comprising contacting the cell with insulin prior to contacting the cell with the composition.
75. The method of Clause 73, wherein the cell is a skeletal muscle cell, a cardiac muscle cell, a smooth muscle cell, an adipose tissue cell, a hippocampal cell, or a cerebellum cell.
76. The method of Clause 73, wherein the cell is a melanoma cell, prostate cancer cell, cancer cell of muscle tissue, such as rhabdomyosarcoma cell, or a breast cancer cell.
77. A method of treating a disease or condition in a subject with a genetic mutation in a gene encoding dystrophin protein, comprising administering to a subject a therapeutically effective amount of a composition of Clause 71.
78. The method of Clause 77, wherein the subject has or is a genetic carrier of Duchenne muscular dystrophy ("DMD") or Becker muscular dystrophy ("BMD").
79. The method of Clause 77, wherein the antibody fragment or antibody derivative is or comprises a single chain antibody, an Fab fragment, an F(ab)2 fragment, a V_{H}H fragment, a V_{NAR} fragment, or a nanobody.
80. The method of Clause 79, wherein the single-chain antibody is a single chain variable fragment (scFv), or a single-chain Fab fragment (scFab).
81. The method of Clause 77, wherein the nucleic acid is in linear form, plasmid form, or minicircle form.
82. The method of Clause 81, wherein the composition further comprises a histone protein or a plurality of histone proteins associated with the nucleic acid.
83. The method of Clause 81, wherein the composition further comprises a liposome associated with the nucleic acid.
84. The method of Clause 77, further comprising a plurality of cationic peptide moieties associated with the nucleic acid, thereby resulting in condensed nucleic acid.
85. The method of Clause 77, wherein the encoded dystrophin protein has at least 80% sequence identity to SEQ ID NO:5.
86. The method of Clause 77, wherein the subject is a human.

## Claims

1. A composition comprising an antibody, or fragment or derivative thereof, that specifically binds to glucose transporter 4 ("GLUT4") protein, and a therapeutic payload conjugated to the antibody, or fragment or derivative thereof,
wherein the antibody, or fragment or derivative thereof, comprises a heavy chain variable region (V_{H}) and a light chain variable region (V_{L}), wherein:
a) the V_{H} comprises a VH-CDR1 having a sequence set forth in SEQ ID NO: 12, a VH-CDR2 having a sequence set forth in SEQ ID NO:20, and a VH-CDR3 having a sequence set forth in SEQ ID NO: 28; and
b) the V_{L} comprises a VL-CDR1 having a sequence set forth in SEQ ID NO:36, a VL-CDR2 having a sequence set forth in SEQ ID NO:44, and a VL-CDR3 having a sequence set forth in SEQ ID NO:52.

2. The composition of Claim 1, wherein:
(a) the antibody is a monoclonal antibody;
(b) the antibody fragment or antibody derivative is or comprises a single chain antibody, an Fab fragment, an F(ab)2 fragment, a V_{H}H fragment, a V_{NAR} fragment, or a nanobody, optionally wherein the single-chain antibody is a single chain variable fragment (scFv), or a single-chain Fab fragment (scFab);
(c) the antibody, or fragment or derivative thereof, is biotinylated; or
(d) the composition further comprises albumin, optionally wherein at least a portion of the albumin is biotinylated, optionally wherein the antibody, or fragment or derivative thereof, is biotinylated and the composition further comprises neutravidin.

3. The composition of Claim 1 or Claim 2, wherein the therapeutic payload comprises a nucleic acid, a protein or peptide, a lipid, a small molecule pharmaceutical, or a radioisotope.

4. The composition of any one of the preceding Claims, wherein the therapeutic payload comprises a nucleic acid, wherein the nucleic acid is selected from DNA, mRNA, siRNA, and shRNA.

5. The composition of any one of the Claims 1-3, wherein the therapeutic payload comprises a nucleic acid, wherein the nucleic acid comprises an open reading frame operatively linked to a promoter sequence.

6. The composition of any one of the Claims 1-3, wherein the therapeutic payload comprises a nucleic acid in linear form, a nucleic acid in plasmid form, or a nucleic acid in minicircle form.

7. The composition of Claim 6, wherein the composition further comprises a histone protein or a plurality of histone proteins associated with the nucleic acid, optionally wherein the histone protein or at least a portion of the plurality of histone proteins is biotinylated, optionally wherein the antibody, or fragment or derivative thereof, is biotinylated and the composition further comprises neutravidin.

8. The composition of Claim 6, wherein the composition further comprises a liposome associated with the nucleic acid.

9. The composition of Claim 8, wherein the liposome comprises:
(a) one or more lipids selected from DC-Cholesterol-HCl,3b-[N-(N',N'-dimethylaminoethane)-caramoyl]cholesterol hydrochloride ("DC-Chol"), 1,2-dioleoyl-sn-glycero-3-phosphoethanolamine ("DOPE"), 1,2-di-O-octadecenyl-3-trimethylammonium ("DOTMA"), N-[1-(2,3-Dioleoyloxy)propyl]-N,N,N-trimethylammonium propane ("DOTAP"), dimethyldioctadecylammonium bromide ("DDAB"), 1,2-dimyristoyl-3-trimethylammonium-propane ("DMTAP"), 1,2-dioleoyl-3-dimethylammonium-propane ("DODAP"), cholesteryl hemisuccinate ("CHEMS") and cholesterol ("CHOL"), 2,3-dioleyloxy-N-[2(sperminecarboxamido)ethyl]-N,N-dimethyl-1-propanaminium ("DOSPA"), Di-octadecyl-amido-glycyl-spermine ("DOGS"), dioleoylphosphatidylcholine ("DOPC"), and the like; or
(b) polyethylene glycol ("PEG").

10. The composition of Claim 6, wherein the composition further comprises a plurality of cationic peptide moieties associated with the nucleic acid, thereby resulting in condensed nucleic acid; optionally wherein:
(a) the cationic peptide moieties are or comprise poly-L-Lysine ("PLL"), linear *Polyethylenimine* ("LPEI"), branched *Polyethylenimine* ("BPEI"), chitosan, spermidine and spermine, Polyamidoamine ("PAMAM"), poly(2-dimethylaminoethyl methacrylate) (*"PDMAEMA*")*,* Poly(beta-amino ester)s (*"PBAEs"*)*,* poly {*N*-[*N*-(2-aminoethyl)-2-aminoethyl]aspartamide ("PAsp(DET)"), poly(2-aminoethyl ethylene phosphate ("PPEEA"), and the like; or
(b) at least a portion of the plurality the cationic peptide moieties is biotinylated, optionally wherein the antibody, or fragment or derivative thereof, is biotinylated and the composition further comprises neutravidin.

11. The composition of any one of the Claims 1-3, wherein the therapeutic payload comprises a nucleic acid with a promoter sequence operatively linked to a sequence encoding a dystrophin protein, or a functional fragment thereof, optionally wherein:
(a) the dystrophin protein is a substantially full-length human dystrophin protein; or
(b) the full-length human dystrophin protein has an amino acid sequence with at least 80% sequence identity to the sequence set forth in SEQ ID NO:5.

12. A composition as recited in any one of Claims 1-3 for use in a method of treating a disease or condition in a subject with a genetic mutation in a gene encoding dystrophin protein, wherein the therapeutic payload comprises a nucleic acid with a promoter sequence operatively linked to a sequence encoding a dystrophin protein, or a functional fragment thereof;
wherein the method comprises administering a therapeutically effective amount of the composition to the subject.

13. The composition for use according to Claim 12, wherein the subject has or is a genetic carrier of Duchenne muscular dystrophy ("DMD") or Becker muscular dystrophy ("BMD").

14. An *in vitro* method of targeting a therapeutic payload for delivery to a cell that expresses GLUT4, the method comprising contacting the cell with the composition as recited in any one of Claims 1-3.

15. The method of Claim 14:
(a) further comprising contacting the cell with insulin prior to contacting the cell with the composition;
(b) wherein the cell is a skeletal muscle cell, a cardiac muscle cell, a smooth muscle cell, an adipose tissue cell, a hippocampal cell, or a cerebellum cell; or
(c) wherein the cell is a melanoma cell, prostate cancer cell, cancer cell of muscle tissue, such as rhabdomyosarcoma cell, or a breast cancer cell.
